# EUROPEAN PATENT APPLICATION

(11) **EP 1 279 733 A1**
(43) Date of publication of application: **29.01.2003**
(21) Application number: 01401993.9
(22) Date of filing: 24.07.2001
(51) Int. Cl.: C12N 15/12, C07K 14/47, C07K 16/18, C12N 1/21, A61K 31/7088, C12Q 1/68

(54) **Nucleic acids and polypeptides involved in the predisposition to infection to human papilloma virus, to epidermodysplasia verruciformis and/or to psoriasis**

(71) Applicant: INSTITUT PASTEUR, 75724 Paris Cédex 15 (FR); INSTITUT NATIONAL DE LA SANTE ET DE LA RECHERCHE MEDICALE (INSERM), 75654 Paris Cédex 13 (FR)
(72) Inventor: Orth, Gérard, 92330 Sceaux (FR); Favre, Michel, 75015 Paris (FR); Ramoz, Nicolas, 76380 Canteleu (FR)
(74) Representative: Desaix, Anne

(57) **Abstract**

The present invention relates to the Evin-1 and Evin-2 genes, their allelic forms and the polypeptides encoded by these genes involved in the susceptibility to infection with Human Papilloma Virus (HPV), Epidermodysplasia verruciformis (EV), and/or to psoriasis. The invention also relates to the use of Evin-1 and Evin-2 genes and their expression products or fragments thereof for the diagnosis of a susceptibility to HPV, to EV and/or to psoriasis. Finally, the invention relates to pharmaceutical compositions comprising Evin-1 or Evin-2 derived nucleic acids and to the screening of new drugs for the treatment or the prevention of infection to HPV, EV and/or psoriasis.

## Description

The present invention relates to the Evin-1 and Evin-2 genes, their allelic forms and the polypeptides encoded by these genes involved in the susceptibility to infection with Human Papilloma Virus (HPV), Epidermodysplasia verruciformis (EV), and/or to psoriasis. The invention also relates to the use of Evin-1 and Evin-2 genes and their expression products or fragments thereof for the diagnosis of a susceptibility to HPV, to EV and/or to psoriasis. Finally, the invention relates to pharmaceutical compositions comprising Evin-1 or Evin-2 derived nucleic acids and to the screening of new drugs for the treatment or the prevention of infection to HPV, EV and/or psoriasis.

Epidermodysplasia verruciformis (EV) is a rare genodermatosis associated with a high risk of skin cancer (Jablonska et al., 1972; McKusick, 1998). EV results from an abnormal susceptibility to specific related human papillomavirus (HPV) genotypes and to the oncogenic potential of some of them, mainly HPV5 (Orth et al, 1978, 1979; Orth, 1987). Whereas EV-associated HPV (EV HPV) genotypes cause widespread inapparent infections in the general population (Boxman *et al.,* 1997; Astori *et al.,* 1998), infection leads to the early development of disseminated flat wart-like and pityriasis versicolor-like lesions in EV patients. Patients are unable to reject their lesions and cutaneous Bowen's carcinomas *in situ* and invasive squamous cell carcinomas develop in about half of the patients, mainly on sun-exposed areas (Jablonska *et al.,* 1972, Rueda and Rodriguez, 1976; Majewski *et al.,* 1997). Cancers are usually associated with HPV5 and occasionally with HPV types 8, 14, 17, 20, or 47 (Orth, 1987).

A genetic linkage performed on three consanguineous EV families, two originating from Algeria and one from Colombia allowed to map susceptibility locus for EV to a 1 cM (J Megalese pair) interval flanked by D17S939 and D17S802 (Ramoz *et al.,* 1999); then, this interval was reduced to 300 kb between 804cd and D17S802 (Ramoz, Thèse universitaire, 2000). This locus is known as the EV1 locus. The EV1 locus represents the first locus known so far for a gene controlling the susceptibility to an oncogenic HPV genotype. Using homozigosity mapping strategy (Lander and Botstein, 1987), a second EV susceptibility locus (EV2) have been mapped to the 2p21-p24 chromosomal region (Ramoz et al., 2000). These results provide evidence for a nonallelic heterogeneity in EV. However, the gene(s) involved in the susceptibility for EV are still unknown. Indeed, numerous genes and EST have been assigned to the 17qter region containing EV1 (Plummer *et al*., 1997). Among these genes, some were likely to play a role in the control of the expression of the biological properties of EV HPV, such as those encoding transcription factors like ILF1 (interleukin enhancer binding factor 1) or proteins involved in signal transduction like GRB2 (growth factor receptor binding protein 2) or the protein-kinase C α subunit. None of them, however have been mapped accurately.

Actually, *loci* for the susceptibility to different skin diseases have been mapped to region 17qter. A susceptibility locus for nonepidermolytic palmoplantar keratoderma associated with oesophageal cancer has been mapped between the D17S1839 and D17S1603 markers (Kelsell *et al.,* 1996), centromeric to EV1. A frequent loss of heterozygosity of the D17S785 marker located close to EV1 has been reported in actinic keratoses and squamous cell carcinomas of the skin (Quinn et al., 1994; Rehmann et al., 1996), suggesting that this region contains a gene important in the development of squamous neoplasia. A dominant susceptibility locus for familial psoriasis has been recently mapped near the D17S802 marker, *i.e.,* between D17S802 and D17S928 (Tomfohrde et al., 1994) and between D17S785 and D17S802 markers (Nair et al., 1997). This region contains EV1.

It is worth stressing that patients suffering from psoriasis have recently been shown to constitute the long-searched for reservoir of HPV5, the genotype associated with EV carcinomas (Favre et al., 1998, Majewski et al., 1998). Using nested polymerase chain reaction methods, HPV5 was detected in more than 90% of patients with psoriasis, in contrast with the rare detection of this particular EV HPV genotype in skin specimens from normal individuals or patients with skin cancers (Boxman *et al.,* 1997; Pfister and ter Schegget, 1997; Astori *et al.,* 1998). Furthermore, antibodies against HPV5 capsid proteins were found in a significant proportion of psoriatic patients, which indicates the occurrence of a complete HPV5 life cycle in some epidermal cells (Favre *et al.,* 1998; Majewski *et al.,* 1998). This suggests that the host restriction towards HPV5 infection is somewhat less efficient in patients suffering from psoriasis than in the general population, and raises the question of the role of HPV5 in the immunopathogenesis of psoriasis (Majewski *et al.,* 1998). Patients suffering from psoriasis or EV are not known to be prone to EV or psoriasis, respectively, however these data suggest that distinct defects affecting the same gene on chromosome 17qter may be involved in the two skin conditions.

In the present invention, the bioinformatic analysis of the sequence contained in the EV1 locus, the identification of novel polymorphic markers in this region and genetic analysis from haplotype segregation allowed to further delimit the EV1 locus to a 180 kb region between 804EF and 532AB.

It has been surprisingly found that the susceptibility to EV mapping to the EV1 locus is related to abnormal expression of any of two novel genes which have been named Evin-1 and Evin-2. More specifically, by sequencing four ORFs, it was discovered that the presence of non-sense mutations in the two novel ORFs, Evin-1 and Evin-2, correlated to a susceptibility to EV.

Taken into consideration the relationship identified between susceptibility to HPV infection, EV and psoriasis, these new genes can serve as the basis for development of diagnostic tests, such as those capable of predicting the susceptibility to HPV infection, EV and psoriasis. These genes also provide new means, either directly or indirectly, for identifying drugs involved in the treatment or the prevention of HPV infection, EV and psoriasis.

cDNAs derived from Evin-1 and Evin-2 genes have been purified and sequenced. Such sequences of cDNA of Evin-1 and Evin-2 are shown in SEQ ID NO:1 and SEQ ID NO:2, respectively. The complete sequences of the corresponding new genes Evin-1 and Evin-2 related to susceptibility to HPV-infection, EV, or psoriasis are shown in SEQ ID NO:3 and SEQ ID NO:4.

As used herein the term "HPV infection" relates to infection with any HPV, including either cutaneous or genital type HPV. Especially, it relates to HPV type 5 infection, but also to HPV types 8, 14, 17, 20, 47, 33, 32, 34, 36, 39, 42 and phylogenetically related types.

Naturally, polymorphic variants of any of SEQ ID NOs 1-4 may be purified by the one skilled in the art by hybridizing a fragment of SEQ ID NO: 1-4 to genomic DNA libraries or cDNA libraries and isolating the corresponding hybridizing nucleic acid from said DNA or cDNA libraries for example.

Thus, one first aspect of the invention is a purified nucleic acid, characterized in that it hybridizes to a nucleotide sequence selected among SEQ ID NO:1, NO:2, NO:3 and NO:4, under stringent conditions.

As used herein, the term "nucleic acid" refers either to a genomic DNA, a cDNA or a messenger RNA (mRNA). It can be either a single-stranded or a double-stranded nucleic acid.

The length of the nucleic acids of the invention depends upon their application. For use as a probe for hybridization to genomic DNA or cDNA libraries, in molecular diagnosis, such as Southern Blot or Northern Blot, for example, fragments have a length generally comprised between 100 and 250 bp.

In one embodiment, the nucleic acid of the invention is at least 15 nucleotides long.

For use, as a primer for PCR amplification for example, preferred fragments are those having a length comprising between 15 nucleotides to 35 nucleotides, or around 20 nucleotides.

The invention also relates to a mixture of fragments of nucleic acids encompassing fragments of any of SEQ ID NO: 1, 2, 3 and 4, said fragments having a length generally comprised between 100 and 250 bp.

As used herein, "stringent conditions" are selected among the conditions which enable specific hybridization of two single strand DNA at around 65°C, for example, in a solution of 6X SSC, 0.5% SDS, 5X Denhardt's solution and 100 mg of non specific carrier DNA, or any other solution with equivalent ionic strength. Parameters defining the stringency conditions depend upon the melting temperature (Tm). For sequences up to 30 bp, Tm is defined as follows: Tm=81.5+0.41(%G+C)+16.6Log (concentration of cations) - 0.63(% formamide) - (600/number of bases). For the sequence less than 30 bp, Tm is defined as follows: Tm=4(G+C) + 2(A+T). The stringent conditions can thus be adapted by the one skilled in the Art, depending upon the length of the nucleic acid, the GC content and/or any parameters, according especially to the protocols as defined by Sambrook *et al.,* 2001 (Molecular Cloning: A Laboratory Manual, 3^{rd} Ed., Cold Spring Harbor, laboratory press, Cold Spring Harbor, New York).

The nucleic acids of the invention can be purified by any means known in the art.

As used herein, The terms "isolated" and "purified" according to the invention refer to a level of purity that is achievable using current technology. The molecules of the invention do not need to be absolutely pure (i.e., contain absolutely no molecules of other cellular macromolecules), but should be sufficiently pure so that one of ordinary skill in the art would recognize that they are no longer present in the environment in which they were originally found (i.e., the cellular medium). Thus, a purified or purified molecule according to the present invention is one that has been removed from at least one other macromolecule present in the natural environment in which it was found.

The molecules of the invention can be essentially purified and/or purified, which means that the composition in which they are present is almost completely, or even absolutely, free of other macromolecules found in the environment in which the molecules of the invention are originally found. Isolation and purification thus does not occur by addition or removal of salts, solvents, or elements of the periodic table, but must include the removal of at least some macromolecules.

For short fragments, and especially those inferior to 50 nucleotides and single stranded, chemical synthesis can also be used. For longer fragments, the nucleic acids of the invention are amplified according to amplification methods such as Strand Displacement Amplification (SDA) technique (Walker et al, 1992, Nucleic Acids Res., 1691-1696) or PCR, with primers selected from the disclosed sequences of any of SEQ ID NOs 1-4. They can also be obtained from culturing the strain deposited at the C.N.C.M on the 18^{th} of July 2001, under accession number 1-2704, recovering the plasmid containing the nucleic acids of the invention, digesting the plasmid with appropriate restriction enzymes and purifying the desired restriction fragments according to usual methods of molecular biology. The nucleic acids of the invention can also be obtained using conventional techniques of screening of available human libraries, using a fragment of a nucleic acid of SEQ ID NO 1, NO:2, NO:3 or NO:4.

In one embodiment, the purified nucleic acid of the invention is selected among
a) Evin-1 cDNA as shown in SEQ ID NO:1 or Evin-1 gene as shown in SEQ ID NO:3;
b) Evin-2 cDNA as shown in SEQ ID NO:2 or Evin-2 gene as shown in SEQ ID NO:4;
c) a fragment of a nucleic acid selected among SEQ ID NO:1, NO:2, NO:3 and NO:4;
d) a mutated fragment of a nucleic acid selected among SEQ ID NO:1, NO:2, NO:3 and NO:4, said mutated fragment having at least one point mutation with respect to the corresponding wild type fragment.

As used herein, the term "fragment of a nucleic acid selected among SEQ ID NO:1, NO:2, NO:3 and NO:4" refers to a nucleic acid which is identical or which does not hybridize under stringent conditions to sequences of the human genome others than those selected among SEQ ID NO:1, NO:2, NO:3 and NO:4 or fragments thereof.

As used herein, a point mutation is a mutation which consists of the modification of one nucleotide to another. For a clear understanding of the designed mutation, the point mutation will be indicated as follows:
"X----Y", wherein "X" represents the type of nucleotide A, C, G or T which is present in the wild type nucleic acid; "----" represents the position of the mutated nucleotide located in a specific ORF, and "Y" represents the type of the mutated nucleotide, A, C, G or T, in the mutated nucleic acid.

More specifically, a preferred embodiment of the above-defined nucleic acid of the invention is characterized in that it consists of a mutated fragment from SEQ ID NO:1, said mutated fragment having one of the following point mutations: C280T, G1726T, wherein 280 or 1726 refers to the position of the mutated nucleotide in SEQ ID NO:1.

Mutated fragments can be purified from a patient suffering from EV. It can also be obtained by site-directed mutagenesis of the corresponding wild-type nucleic acid using methods well-known in the Art.

Examples of such mutated fragments are fragments located around positions 280 or 1726 in SEQ ID NO:1. Indeed, these fragments correspond to fragments of the Evin-1 gene harbouring non-sense mutations which have been shown to be related to a susceptibility for EV.

The complete cDNA sequence of Evin-1 having the first mutation C280T is shown in SEQ ID NO:7. The complete cDNA sequence of Evin-1 having the point mutation G1726T is shown in SEQ ID NO:8.

Accordingly, another embodiment of a nucleic acid of the invention is further characterized in that it is selected among SEQ ID NO:7, SEQ ID NO:8 or fragments thereof.

Another embodiment of the above-defined nucleic acid of the invention is characterized in that it consists of a mutated fragment of SEQ ID NO:2, having at least the following point mutation: G1129T, wherein 1129 correspond to the position of the mutated nucleotide in SEQ ID NO:2.

Examples of such mutated fragments are fragments located around positions 1129 in SEQ ID NO:2. Indeed, these fragments correspond to fragments of the Evin-2 gene harbouring non-sense mutations which have been shown to be related to a susceptibility for EV.

The complete cDNA sequence of Evin-2 having the point mutation G1129T is shown in SEQ ID NO:9.

Accordingly, another specific embodiment of the nucleic acid of the invention is further characterized in that it consists of SEQ ID NO:9 or a fragment thereof.

It has been shown that the detected mutations correlated to a susceptibility to EV. Indeed, these mutations corresponding to non-sense mutations appear to be responsible for abnormal expression of the Evin-1 or Evin-2 gene.

In another embodiment, the purified nucleic acid of the invention is further characterized in that it encodes a polypeptide involved in the susceptibility for developing Epidermodysplasia verruciformis.

By "susceptibility" used herein is meant that the subject is prone to the specific infection. Therefore, the subject has an increased probability for developing the disease with respect to the general population.

In another embodiment of the invention, the nucleic acid of the invention comprises a mutated gene whose expression is correlated to infection with human papillomavirus susceptibility to infection to Human Papilloma Virus (HPV), to Epidermodysplasia verruciformis, and increase the predisposition to skin diseases such as psoriasis.

Examples of said nucleic acid comprising a mutated gene are the nucleic acids comprising the Evin-1 gene having one of the following mutations:
C280T (changing an arginine codon into a stop codon) and G1726T (changing a glutamic acid codon into a stop codon).

Another example is the one comprising the Evin-2 gene having the following mutation: G1129T (changing a glutamic acid codon into a stop codon).

It is another object of the invention to provide the expression products of the Evin-1 or Evin-2 genes or their derivatives. These expression products can be used for screening new drugs for treating or preventing HPV infection, EV, and/or psoriasis. These expression products also provide new means for the preparation of antibodies useful, for example, in molecular diagnosis of the susceptibility to HPV infection, EV, and/or psoriasis.

Thus, the invention also provides an purified polypeptide characterized in that it is encoded by any of the nucleic acid of the invention encoding a polypeptide involved in the susceptibility for developing Epidermodysplasia verruciformis as mentioned above.

One polypeptide of the invention is the Evin-1 protein as shown in SEQ ID NO:5.

Another polypeptide of the invention is the Evin-2 protein as shown in SEQ ID NO:6.

In yet another embodiment, the purified polypeptide of the invention is characterized in that it consists of a truncated form of SEQ ID NO:5 at amino-acid positions 94 or 576 (SEQ ID NO:10¹ or SEQ ID NO:11 respectively), or of a truncated form of SEQ ID NO:6 at amino acid position 377 (SEQ ID NO:12), the expression of said truncated form being involved in an increased susceptibility to infection to Human Papilloma Virus (HPV), to Epidermodysplasia verruciformis, and/or to skin diseases such as psoriasis in human.

The polypeptide of the invention can be produced as a recombinant polypeptide in a cellular expression system or as a synthetic polypeptide in a cell-free expression system. The one skilled in the Art will know how to select the appropriate expression system.

Optionally, the polypeptides of the invention may be fused to another polypeptide, especially for convenient expression and/or purification from an expression system. They can be, for example, fused to an amino acid sequence containing any signal necessary for protein stability, protein secretion and/or protein purification.

The polypeptides of the invention can be produced using a cellular expression system.

The polypeptides according to the invention can also be prepared by conventional method of chemical synthesis, either in a homogenous solution or a solid phase. As an illustrative embodiment of such chemical polypeptide synthesis techniques the homogenous solution technique described by Houbenweyl *et al.* in 1974 may be cited.

The polypeptide can be characterized by binding onto an immunoaffinity chromatography column on which polyclonal or monoclonal antibodies directed to the polypeptide or fragments have been previously immobilized by a method well known by the person skilled in the art.
Naturally, every variants of polypeptides comprising SEQ ID NO: 5 or SEQ ID NO: 6 are included in the present invention.
A polypeptide "variant" as referred to herein means a polypeptide substantially homologous to native Evin-1 or Evin-2 polypeptides, but which has an amino acid sequence different from that of native polypeptides because of one or more deletions, insertions, or substitutions. The variant amino acid sequence preferably is at least 80% identical to a native Evin-1 or Evin-2 polypeptides amino acid sequences, or at least 90% identical. The percent identity can be determined, for example, by comparing sequence information using the GAP computer program, version 6.0 described by Devereux et al. (Nucl. Acids Res. 12:387, 1984) and available from the University of Wisconsin Genetics Computer Group (UWGCG). The GAP program utilizes the aligment method of Needleman and Wunsch (J. Mol. Biol. 48:443, 1970), as revised by Smith and Waterman (Adv. Appl. Math 2:482, 1981). The preferred default parameters for the GAP program include: (1) a unary comparison matrix (containing a value of 1 for identities and 0 for non-identities) for nucleotides, and the weighted comparison matrix of Gribskov and Burgess (Nucl. Acids Re. 14:6745, 1986) as described by Schwartz and Dayhoff, eds., (Atlas of Protein Sequence and Strucutre, National Biomedical Search Fundation, pp. 353-358, 1979) (2) a penalty of 3.0 for each gap and an additional 0.10 penalty for each symbol in each gap; and (3) no penalty for end gaps
The polypeptides of the invention can be labeled. In one embodiment, the labeled polypeptides are in purified form. Furthermore, the unlabeled or labeled polypeptide is capable of being immunologically recognized by human body fluid containing antibodies. The polypeptides can be labeled, for example, with an immunoassay label selected from the group of radioactive, enzymatic, fluorescent, chemiluminescent labels, chromophores and the like.

As a consequence, another aspect of the invention relates to a recombinant vector characterized in that it comprises a nucleic acid of the invention.

As used herein, the term "vector" includes any type of vector enabling the introduction of a nucleic acid and optionally, the expression of the polypeptide encoded by said nucleic acid in a host cell.

The vector can comprise sequences appropriate for gene expression such as promoter and terminator sequences. Such expression vectors include, among others, chromosomal, episomal or virus-derived vectors, vectors derived from bacterial plasmids, phages, transposons, plasmids and yeast chromosomes, viruses such as baculovirus or papovirus and SV40, adenoviruses and other retroviruses, and combinations thereof, especially, cosmids and phagemids.

In another embodiment, the recombinant vector of the invention is characterized in that it is a plasmid comprising a nucleic acid of the invention, said nucleic acid encoding a polypeptide involved in the susceptibility for developing Epidermodysplasia verruciformis and said plasmid further comprising appropriate sequences for expression of said polypeptide in a host cell. Yet another embodiment of the vector according to the invention is a vector appropriate for expression in a mammalian cell.

The invention also relates to a host cell characterized in that it is transformed by any of the vectors of the invention as defined above.

In one embodiment a host cell is characterized in that it is a bacteria, preferably, an *E. coli* cell. Transforming bacterial cells with the recombinant vectors of the invention is useful for amplification and production and/or storage of the nucleic acids of the invention.

An example of a host cell is the bacterial strain as deposited at the C.N.C.M on the 18^{th} of July 2001, under accession number 1-2704.

Host cells for expression can also be selected among eukaryotic host cells, especially yeast cells such as *Saccharomyces cerevisiae,* filamentous fungi such as *Aspergillus,* insect cells such S2 cells from *Drosophila* or Sf9 from *Spodoptera,* or even plant cells.

Another host cell of the invention is characterized in that it is an eukaryotic cell, for example, a mammalian cell, or a human cell.

A host cell that can be used as a model for analysing the role of Evin-1 and Evin-2 genes and polypeptides encoded by these genes in the development of EV, psoriasis or HPV-associated diseases, is a cell derived from skin tissue and from the epidermis, such as a keratinocyte, a melanocyte or a Langerhans cell.

The host cells of the invention may also be infected by Human Papilloma Virus, especially by HPV type 5.

The invention also concerns an antibody characterized in that it specifically recognizes an purified polypeptide of the invention. These antibodies are useful in the detection and/or the quantification of the expression of normal/abnormal Evin-1 or Evin-2 protein in human biological sample, for example, for diagnosis purposes.

The invention relates to both monoclonal or polyclonal antibodies.

Polyclonal antibodies can be obtained by immunizing an appropriate host with one polypeptide of the invention or fragments thereof containing the epitope(s). The antibodies produced from a serum of an immunized host are then recovered, for example, by contacting the sera with purified polypeptides and recovering the antibodies forming an antigen-antibody reaction.

Monoclonal antibodies according to the invention are obtained according to the classical method of cultures of hybridomas described by Kohler et Milstein (1975).

Antibodies of the invention may be coupled to another compound, such as a marker, in order to obtain a detectable or quantifiable signal.

Expression of a truncated forms of the Evin-1 or Evin-2 protein is correlated to a susceptibility to EV, to psoriasis and/or to infection to Human Papilloma Virus. Thus, an antibody specifically directed to the C-terminal part of the Evin proteins which is absent in the truncated form would be useful to detect selectively the wild type form of the protein.

Therefore, in one specific embodiment, an antibody of the invention is characterized in that it is specifically directed against the C-terminal part of SEQ ID NO:5 starting from amino acid position 94 or from amino acid position 576.

In another specific embodiment, an antibody is characterized in that it is specifically directed against the C-terminal part of SEQ ID NO:6 starting from amino acid position 377.

The nucleic acids of the invention can be used for the preparation of a composition appropriate to modulate the expression of the Evin-1 or Evin-2 gene, either in normal or abnormal forms. The modulation of the expression Evin-1 or Evin-2 gene, compared to the expression of a normal form provides a therapeutic treatment for psoriasis, EV and/or HPV infection.

Thus, the invention relates to the use of a nucleic acid according to the invention for the preparation of a pharmaceutical composition appropriate for treating or preventing Human Papilloma Virus infection, Epidermodysplasia verruciformis and/or psoriasis.

The invention also relates to a pharmaceutical composition comprising a nucleic acid of the invention and a pharmaceutically acceptable vehicle.

More specifically, the pharmaceutical composition comprises a nucleic acid encoding the Evin-1 gene or Evin-2 gene selected among SEQ ID NO:1, NO:2, NO:3 or NO:4.

The above described pharmaceutical compositions can be administered by any route such as orally, systemically, intravenously, intramuscularly, intradermally, mucosally, encapsulated, using a patch and the like. Any pharmaceutically acceptable carrier or adjuvant can be used in this pharmaceutical composition.
Delivery of the therapeutic nucleic acid into a patient may be direct *in vivo* gene therapy (*i.e.,* the patient is directly exposed to the nucleic acid or nucleic acid-containing vector) or indirect *ex vivo* gene therapy (i.e., cells are first transformed with the nucleic acid *in vitro* and then transplanted into the patient).

A pharmaceutically acceptable vehicle can be selected among those known in the field of genetic therapy. Indeed, an appropriate vehicle should target the nucleic acid of the invention to specific tissues, especially skin tissue, allowing the nucleic acid to penetrate into the target cells and to integrate into the genome for expression of a Evin-1 or Evin-2 protein or a specific fragment thereof, therefore, restoring the activity of a deficient Evin-1 or Evin-2 protein in said target cells.

For example for *in vivo* gene therapy, an expression vector containing the nucleic acid is administered in such a manner that it becomes intracellular; i.e., by infection using a defective or attenuated retroviral or other viral vectors as described, for example in U.S. Patent 4,980,286 or by Robbins et al, Pharmacol. *Ther.,* **80** No. 1 pgs. 35-47 (1998).

The various retroviral vectors that are known in the art are such as those described in Miller *et al.* (*Meth. Enzymol.* **217** pgs. 581-599 (1993)) which have been modified to delete those retroviral sequences which are not required for packaging of the viral genome and subsequent integration into host cell DNA. Also adenoviral vectors can be used which are advantageous due to their ability to infect non-dividing cells and such high-capacity adenoviral vectors are described in Kochanek *(Human Gene Therapy,* **10,** pgs. 2451-2459 (1999)). Chimeric viral vectors that can be used are those described by Reynolds *et al. (Molecular Medecine Today,* pgs. 25-31 (1999)). Hybrid vectors can also be used and are described by Jacoby *et al.* (*Gene Therapy,* **4,** pgs. 1282-1283 (1997)).

Direct injection of naked DNA or through the use of microparticle bombardment (e.g., Gene Gun®; Biolistic, Dupont) or by coating it with lipids can also be used in gene therapy. Cell-surface receptors/transfecting agents or through encapsulation in liposomes, microparticles or microcapsules or by administering the nucleic acid in linkage to a peptide which is known to enter the nucleus or by administering it in linkage to a ligand predisposed to receptor-mediated endocytosis (See Wu & Wu, J. Biol. Chem., 262 pgs. 4429-4432 (1987)) can be used to target cell types which specifically express the receptors of interest.

Another application of the nucleic acids of the invention is their use for screening new molecules having a therapeutic activity against human papillomavirus infection, Epidermodysplasia verruciformis and/or psoriasis.

Indeed, the invention provides a method of screening molecules having a therapeutic activity against human papillomavirus infection, Epidermodysplasia Verruciformis and/or psoriasis, said method comprising:
a) culturing eukaryotic cells transformed by the nucleic comprising the Evin-1 gene having one of the following mutations: C280T (changing an arginine codon into a stop codon) and G1726T (changing a glutamic acid codon into a stop codon), or the Evin-2 gene having the following mutation: G1129T (changing a glutamic acid codon into a stop codon);
b) contacting said host cells with molecules having a therapeutic activity against human papillomavirus infection, Epidermodysplasia verruciformis or psoriasis; and
c) selecting the molecule(s) which restore(s) the expression of the wild type gene.
In one embodiment, the selected molecules in step c) are those which restore the transcription and/or the translation of the wild type gene.

The invention also relates to a method for identifying an allelic form of Evin-1 or Evin-2 gene comprising:
a) isolating a nucleic acid which hybridizes to anyone of SEQ ID NOs :1-4 under stringent conditions from a biological sample of a patient having an increased susceptibility to infection to HPV or Epidermodysplasia Verruciformis;
b) sequencing the purified nucleic acid; and
c) detecting a mutation by comparing the novel sequenced nucleic acid with the corresponding wild-type sequence, wherein said mutation indicates an allelic form of Evin-1 or Evin-2 gene.

The invention relates to the use of a nucleic acid to detect *in vitro* Human Papilloma Virus infection, Epidermodysplasia verruciformis, and/or skin diseases such as psoriasis.

Thus, the present invention relates to a method for detecting predisposed infection to HPV, Epidermodysplasia verruciformis, and/or a skin disease such as psoriasis, said method comprising:
a) extracting genomic DNA or RNA from a biological sample; and,
b) detecting in either Evin-1 or Evin-2 gene, specific mutations involved in an increased susceptibility to infection to HPV, to Epidermodysplasia verruciformis, and/or to skin diseases such as psoriasis.

Any methods appropriate for the detection of single mutation can be used for carrying out the above-defined method. Detection of specific mutations in Evin-1 or Evin-2 gene can be performed, for example, by the amplification of a region containing said mutation in Evin-1 or Evin-2, and the sequencing of the amplified product. Among the nucleic acids of the invention, those having a length comprised between 15 and 35 nucleotides and having a sequence located 50-500 bp downstream or uptream the position of the specific mutations, are appropriate for use as primers for amplification of the region to sequence.

Single mutations can also be detected by the use of a DNA chip comprising short primers corresponding to fragments of the Evin-1 or Evin-2 with the specific mutations, and the hybridization of the DNA chip with at least a probe consisting of a fragment of the Evin-1 or Evin-2 gene susceptible to contain the specific mutations to detect. The primers and the conditions for hybridization must be selected in such a way that a single mutation between the probe and one target primer is sufficient to detect a decrease in hybridization of the probe to the target.

The invention also concerns a DNA chip characterized in that it contains at least a nucleic acid according to the invention.

As used herein, a "DNA chip" refers to a support enabling the stable binding of an amount of nucleic acids, the hybridization of said stably bound nucleic acids to a probe and the quantification of the hybridization complex. Technologies relating to DNA chips are described in particular in Sambrook *et al.,* 2001 (supra).

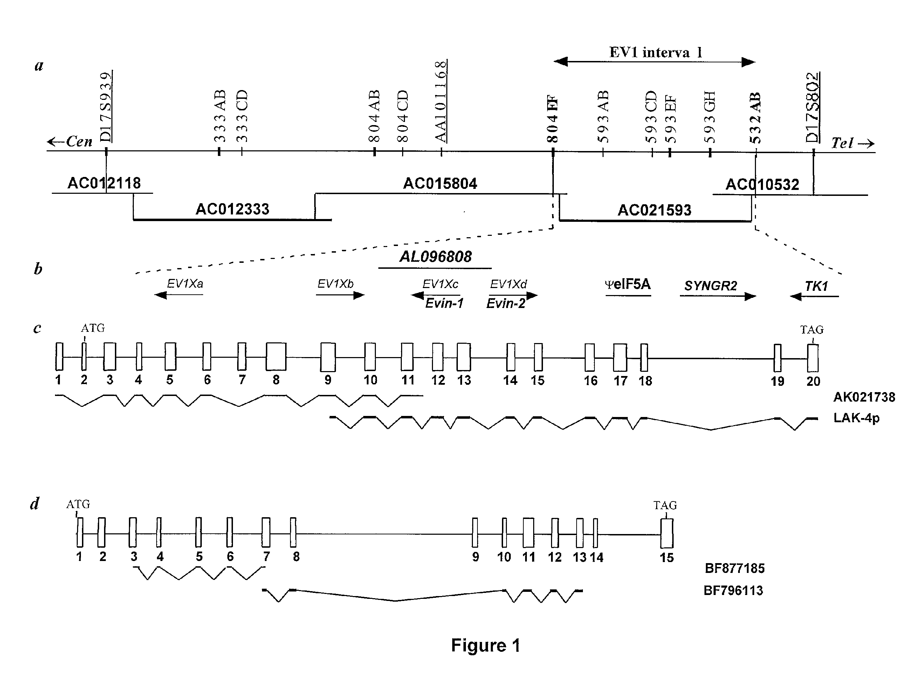

Another method for *in vitro* detection of a susceptibility to infection to HPV, Epidermodysplasia verruciformis, and/or to a skin disease such as psoriasis comprises the use of specific polymorphic markers selected among the following markers: 333AB, 333CD, 804AB, 804CD, 804EF, 593AB, 593CD, 593EF and 532AB as shown in figure 1.

The invention also provides a primer for detecting the novel polymorphic markers, said primer being characterized in that it is selected among any of SEQ ID Nos 22-31.

Another embodiment of the present invention relates to a method for detecting *in vitro* a susceptibility to infection to HPV, to Epidermodysplasia verruciformis, and/or to a skin disease such as psoriasis, in a human biological sample, said method comprising:
a) extracting proteins from a biological sample; and,
b) detecting truncated forms of either Evin-1 or Evin-2 proteins involved in an increased susceptibility to infection to HPV, to Epidermodysplasia verruciformis, and/or to a skin disease such as psoriasis.

The detection of truncated forms of proteins can be carried out by the use of an antibody which specifically recognizes the normal or abnormal form of the Evin-1 or Evin-2 protein. Specific recognition can be detected using ELISA assay or immunoprecipitation assay. Also, one can use a protein chip consisting of antibodies which specifically recognize normal or abnormal forms of Evin-1 or Evin-2 protein, said antibodies being bound to a support at a defined position.

As used herein, a "protein chip" refers to a support enabling the stable binding of an amount of polypeptides, the hybridization of said stably bound polypeptides to a probe and the quantification of the hybridization complex. Technologies relating to protein chips are described, for example, in the following references: Büssow *et al.,* 1998; Emili & Cagney, 2000; MacBeath & Schreiber, 2000; Zhu *et al.,* 2000; Holt *et al.,* 2000, Mahlknecht *et al.,* 2001.

Thus, the invention also provides a protein chip characterized in that it contains at least an antibody according to the invention, or a polypeptide according to the invention.

The invention also concerns the kit for carrying out the methods of invention as defined previously.

Especially, the invention provides a kit for detecting *in vitro* a susceptibility to infection to HPV, to Epidermodysplasia Verruciformis, and/or to a skin disease such as psoriasis, said kit comprising the following components:
a) an purified nucleic according to the invention or a vector according to the invention; and
b) optionally, appropriate buffers for DNA or RNA extraction and/or DNA or RNA hybridization reaction, and/or for detection of the mutated DNA or RNA.

The invention also provides a kit for detecting *in vitro* a susceptibility to infection with HPV, to Epidermodysplasia verruciformis, and/or to skin diseases such as psoriasis, in a human biological sample, said kit comprising the following components:
a) an antibody according to the invention as defined above;
b) optionally, appropriate buffers for protein extraction and/or antigen-antibody immunological reaction, and/or for detection of the antigen-antibody reaction.

The invention also relates to the use of a nucleic acid according to the invention or a protein according to the invention for the screening of proteins involved in the development of HPV-associated disease, EV or psoriasis.

For the screening of proteins involved in the development of HPV-associated disease, EV or psoriasis, one can search for proteins physically interacting with Evin-1 or Evin-2 protein or for proteins which interact with the expression of the Evin-1 gene or Evin-2 gene.

Any method of detecting protein-protein interactions can be carried out for screening such proteins. These methods include for example the yeast two-hybrid system (Vidal & Legrain, 1999; Joung *et al*., 2000; Walhout *et al*., 2000; Uetz *et al.,* 2000) or the phage display (Rebar and Pabo, 1994) or protein chip technologies.

Methods of screening of proteins which interact with the expression of the Evin-1 or Evin-2 gene include the use of DNA chip.

The examples below describes the isolation of the Evin-1 and Evin-2 genes and the detection of specific allelic forms involved in susceptibility to EV. Of course, these examples are only specific embodiments of the invention and do not limit the scope of the claimed invention.

It should be appreciated that the above described cDNA, cDNA fragments, polypeptide, polypeptide fragments, methods, antibodies kits, protein chips and DNA chips are not restricted to human use alone. A person skilled in the art can therefore apply the above to other mammalian species.

### LEGENDS OF THE FIGURES

**Figure 1** is a physical map illustrating the genomic organization of the EV1 locus. **a.** is a physical map of the EV1 region. Anchored markers used to construct the contig of overlapping BACs are underlined. The ten novel microsatellites used to narrow the EV1 region are labeled according to the three last numbers of the accession number of the BAC. The critical EV1 interval between markers 804EF and 532AB flanking the BAC 021593 is indicated. **b.** is a physical map which indicates the position of candidate genes for EV in the EV1 interval, as determined by computational analysis. The thymidine kinase (TK1) and synaptogyrin 2 (SYNGR2) genes, four putative novel genes (EV1Xa-d) and a pseudogene (eIF5A) are shown in the chromosomal order. The transcriptional orientation of each gene is indicated by an arrowhead. The EV1Xc gene designated later *Evin1* gene is contained in the PAC clone AL096808. **c,** indicates genomic organization of the Evin1 gene. Exons are depicted by rectangles. The putative start (ATG) and stop (TAG) codons are shown. Common exons between *Evin1* gene and cloned cDNAs AK021738 and LAK-4p are indicated. Lines connecting exons correspond to introns. **d,** indicates genomic organizationof the Evin2 gene. The putative start (ATG) and stop (TAG) codons are indicated. Common exons between Evin2 gene and cloned cDNAs BF877185 and BF796113 are shown.

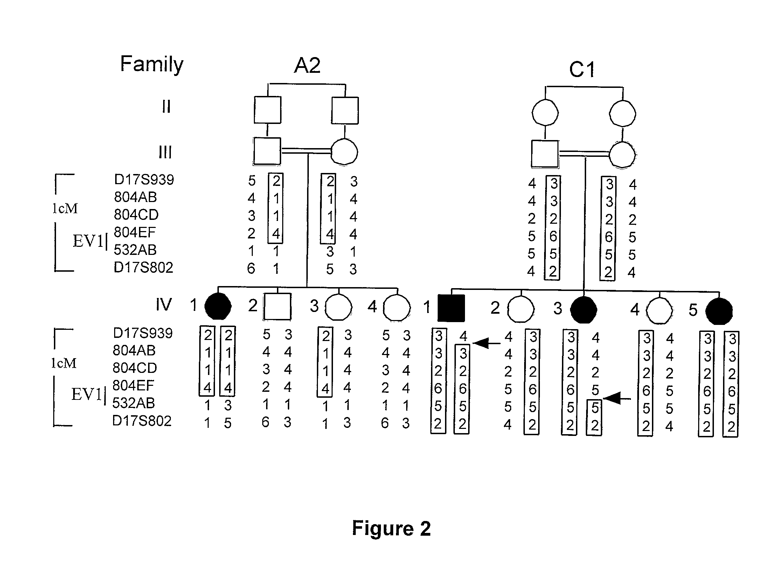

**Figure 2** shows the refinement of the critical region for EV1 on chromosome 17q25. Novel polymorphic markers in the previously mapped 1-centimorgan interval delimited by markers D17S939 and D17S802 are shown on the left. Haplotype segregation and recombination events (arrows) in pedigrees A2 and C1 are shown under the family trees. The disease-associated haplotypes found in an homozygous state in patients (filled circles or rectangles) are boxed.

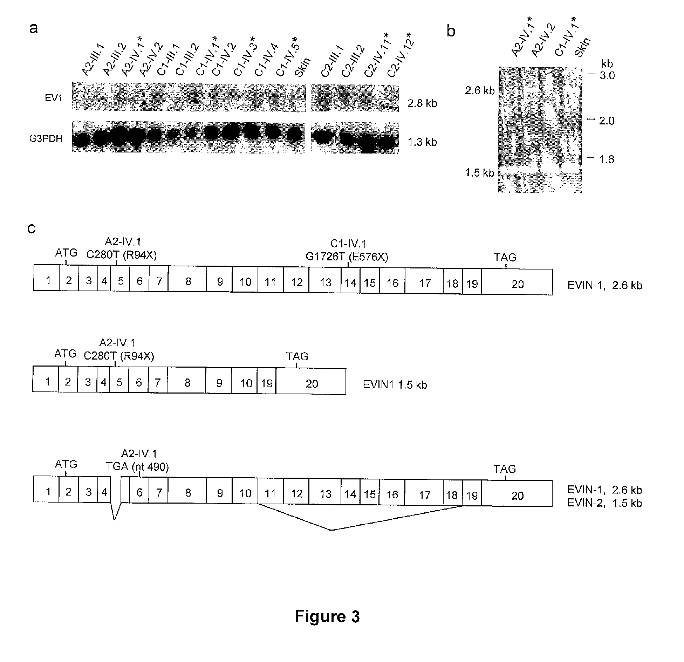

**Figure 3** indicates the expression and structure of *Evin1* transcripts. **a** Northern blot analysis of *Evin1* transcription in lymphoblastoid cell lines of members from pedigrees A2, C1 and C2 or in a normal skin specimen obtained from a mammectomized unrelated patient. Only a 2.8 kb mRNA species is detected using a probe obtained by RT-PCR with primers specific for exons 2 to 9. Rehybridization of the membrane with a GAPDH probe served as the RNA loading control. **b** indicates the amplification of Evin1 cDNAs from total RNA by RT-PCR using primers located in exons 2 and 20 and analysis of products by 1 % agarose gel electrophoresis.Two main cDNAs with sizes corresponding to 2.6 kb and 1.5 kb are detected in lymphoblastoid cells or in the skin. Size markers are indicated on the right. **c** indicates a structure of *Evin1* transcripts. The 2.6 and 1.5 kb cDNAs obtained from the two patients A2-IV.1 and C1-IV.1 and an unaffected sibling A2-IV.2 were cloned and sequenced. Exons 2 to 20 are contained in the 2.6 kb cDNA species while exons 11 to 18 are skipped in the 1.5 kb cDNA species. A C280->T mutation leading to a stop codon (R94->X) are observed in the 2.6 kb and 1.5 kb cDNAs obtained from patient A2-IV.1. In addition, about 50 % of the cDNAs show an alternative splicing resulting in a frameshift mutation in exon 5 (nt 301) and a stop codon in exon 6 (TGA at nt 490). A G1726->T mutation leading to a stop codon (E576->X) is detected in the 2.6 kb cDNA from patient C1-IV.1. X = stop codon.

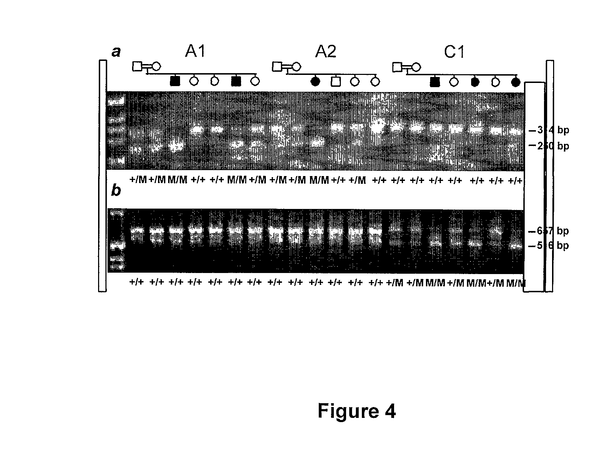

**Figure 4** is a segregation analysis of mutations specific of Evin-1 gene for EV in consanguineous families, **a** The C280->T nucleotide substitution located in Evin-1 gene specific of the disease in the two Algerian pedigrees A1 and A2 creates a *Dde*I restriction site in the exon 5. PCR amplification of sequences encompassing exon 5 generated a 353-bp fragment from the wild type allele which is cleaved into two fragments of 314bp and 39 bp (not shown). The 314 bp fragment is cleaved into two fragments of 250 bp and 64 bp (not shown) in individuals carrying the mutant allele (M). **b** illustrates the G1726->T nucleotide substitution specific of EV in the Colombian pedigree C1 creates a *Mae*I restriction site in the exon 14. Amplification of sequences encompassing the exon 14 generated a 657-bp fragment from the wild type allele which is cleaved into two fragments of 516 bp and 141 bp (not shown) in individuals carrying the mutant allele. Size markers (Gibco BRL) are shown in the first lane.

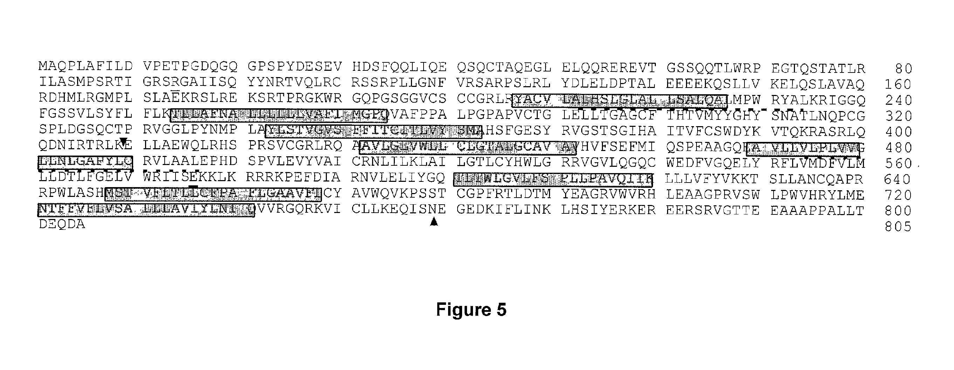

**Figure 5** illustrates the amino acid sequence of the putative proteins encoded by the Evin-1 2.6 kb and 1.5 kb mRNAs. Eight putative transmembrane domains (shaded) and two leucine zipper motifs (underlined) predicted in the 805 amino acid EV1 protein are indicated. The two arrowheads delimits the residues deleted in the 455 amino acid Evin-1 protein. The amino acids found mutated in the families are underlined.

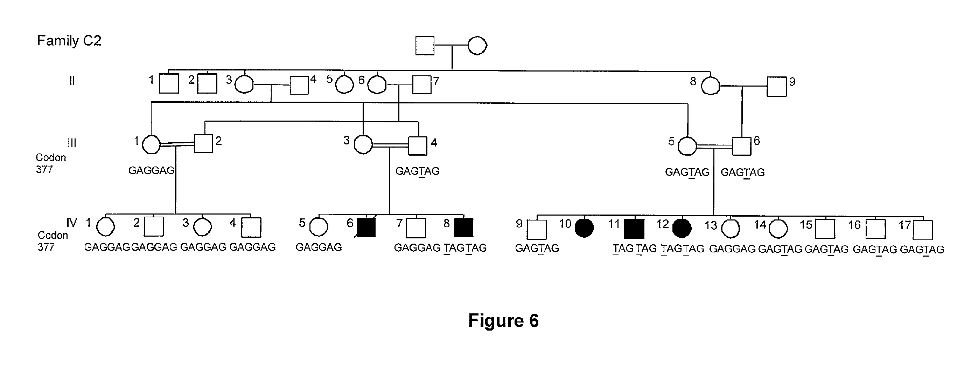

**Figure 6** illustrates the segregation analysis of stop mutation specific of Evin-2 gene for EV in consanguineous family C2. The G1129->T nucleotide substitution located in exon 8 of Evin-2 creates a stop mutation found in an homozygous state in patients C2-IV.8, C2-IV.11,and C2-IV.12.

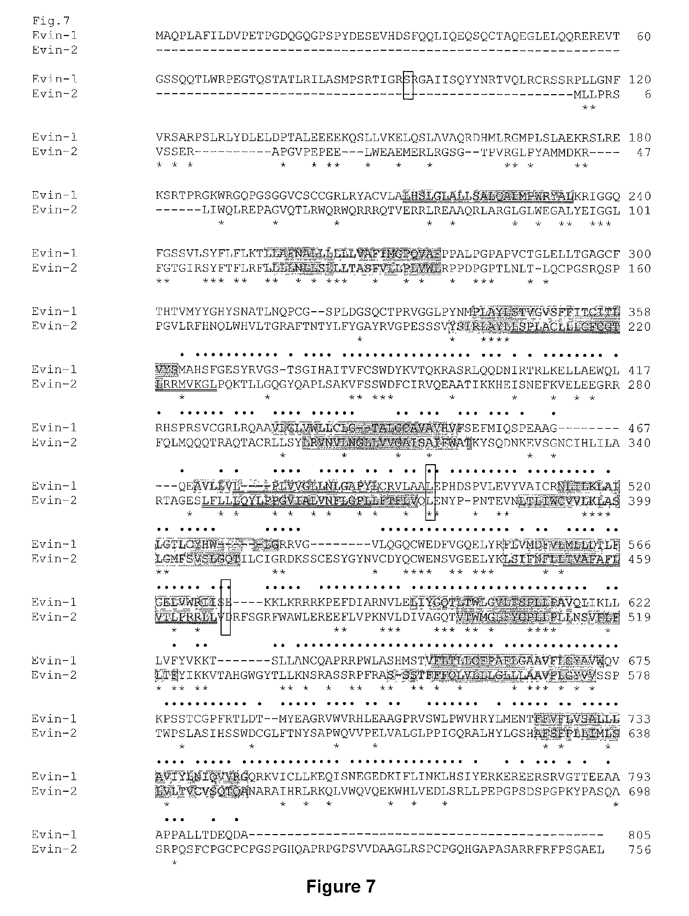

**Figure 7** is an alignment of amino acid sequences of the putative proteins encoded by the Evin-1 and Evin-2 genes. Transmembrane domains (shaded) and leucine zipper motifs (double underlined) predicted in the two proteins are shown. Conserved amino acid are indicated (*). Spots correspond to amino acids conserved between the Evin-1 protein and putative mouse protein BC004840. The amino acids found mutated in Evin-1 (R94 and E576) or Evin-2 (E377) gene are boxed.

### EXAMPLES

### 1/ Refinement of the EV1 locus to a 180 kb region

A first EV1 locus was mapped previously between the D17S939 and D17S802 microsatellite markers (Ramoz *et al.,* 1999); then, this interval was reduced to 300 kb between 804CD and D175802 (Ramoz, Thèse universitaire, 2000). The sequence contigs comprised in the EV1 interval were obtained on the " working draft" sequence available at the Whitehead Institute (http://www-genome.wi.mit.edu).

BLAST alignment analysis (http://www.ncbi.nlm.nih.gov/BLAST/) was used to order the different BAC contigs as shown in Figure 1 from BAC AC012118 to BAC AC010532.
The RepeatMasker program was then used to identify novel putative microsatellites. 10 novel microsatellite markers were identified and positioned on the EV1 locus. The markers were labeled according to the three last numbers of the accession number of the BAC and are also shown in Figure 1.
Four consanguineous EV families comprising a total of 9 patients were studied. Two families originated from Algeria (A1 and A2) and two from Colombia (C1 and C2) (Ramoz *et al.,* 2000).
The patients and their relatives were genotyped as described by *Gyapay et al.* (Gyapay et al, Genotyping procedures in linbroge mapping, Methods, 9: 91-97, 1996), using fluorescence-labeled primers corresponding to the ten putative novel microsatellite markers spanning the EV1 interval. Markers were amplified, separated on an ABI377 DNA sequencer, and analyzed using GENESCAN software (PE-Applied Biosystems).
To map EV1 locus, homozygosity mapping approach was used (Lander and Botstein, Sciences 236: 1567-1570, 1987), this method represents one strategy to map rare human recessive traits in consanguineous families.
Haplotype segregation and recombination events (arrows) in pedigrees A2 and C1 are shown under the family trees in Figure 2.
The results refined the EV1 locus to a 180 kb region as shown in the Figure 1 as the EV1 interval.

### 2/ Identification of known ORFs and putative ORFs in the EV1 interval

Then, bioinformatic analysis using GENSCAN, FGENES and BLAST programs were used to detect known genes, putative novel genes and matching ESTs residing in the refined EV1 interval. More specifically, the EV1 interval region comprises two already known genes, the thymidine kinase (TK1) and synaptogyrin 2 (SYNGR2) genes. The region also comprises four putative novel genes (EV1Xa-d) and one pseudogene (eIF5A). The position of each identified ORF is also shown in Figure 1. The complete sequence of the Evin-1 gene is shown in SEQ ID NO:3. The complete sequence of the Evin-2 gene is shown in SEQ ID NO:4.

### 3/ Mutation detection and confirmation of their specificity for susceptibility to EV

Mutations were searched by first sequencing all the exons of TK1, SYNGR2, and EVIN2 genes and exons 2 to 20 of EVIN1 gene. Exons were amplified by PCR from genomic DNA, using primers located in flanking sequences. Fluorescent DNA sequencing was carried out using BigDye terminator chemistry (PE-Applied Biosystems). Mutations in the Evin-1 gene were found in exon 5 (C280T) in families A1 and A2 or in exon 14 (G1726T) in family C1 which created a new *Dde*I or *Mae*I restriction site, respectively. Mutation in the Evin-2 gene was found in exon 8 (G1129T) in family C2.
In order to confirm that the identified mutations were not due to errors in sequencing, amplification products encompassing exon 5 or exon 14 were obtained by PCR from genomic DNA and treated with *Ddel* (Promega) or *Mael* (Boehringer) restriction endonuclease, as recommended by the supplier. The polymorphism of restriction sites allow the specific detection of mutations and segregation analysis of the identified mutations in consanguineous families.
Digestion products were separated by electrophoresis on 3 % agarose gels and stained with ethidium bromide. Restriction fragments corresponding to *Ddel* or *Mae*l from DNA of individuals having a mutant allele confirmed the presence of a specific mutation in exon 5 of the Evin-1 gene or exon 14 of the Evin-1 gene (see Figure 4a and 4b). The G1129T nucleotide substitution located in exon 8 of Evin-2, creating a stop mutation found in a homozygous state in C2 patients (Figure 6).

### 4/ Northern blot analysis and RT-PCR for isolation of cDNA encoding EVIN-1 and EVIN-2 proteins

Lymphoblastoid cell lines from members of A1, C1, and C2 EV families were established and a normal breast skin specimen was obtained upon mammectomy from an unrelated non EV patient. Total RNA was extracted from cells and specimen using RNeasy Midi kit (Qiagen). RNAs were separated by formaldehyde agarose gel electrophoresis and blotted onto Hybond-C extra membrane (Amersham Pharmacia Biotech) (Sastre Garau 2000). Four blots were hybridized with probes specific for the TK1, SYNGR2, EVIN1 or EVIN2 gene and after stripping rehybridized with a probe specific for the GADPH gene, as control. As shown in Figure 3, when using a probe obtained by RT-PCR with primers specific for exons 2 to 9 of Evin-1, only a 2.8 kb mRNA species was detected.
Total RNA (1 µg) from the unaffected subject A2-IV.2 was reverse transcribed and amplified by PCR, using the enhanced avian RT-PCR kit (Sigma). The forward primers contained the initiation codon of the four genes (TK1-ATG 5'-AGGCGCAATGAGCTGCATTAACCT-3'; SYNGR2-ATG 5'-GCGACGGCGACATGGAGAGC-3'; EVIN1-ATG 5'-AGACTAGCGGGCATTGGCCAGAGACATG-3'; and EVIN2-ATG 5'-CTGGCCCGCCGAGATGCTGCTG-3'). The reverse primers encompassed the junction of exons 8 and 9 for the EVIN1 gene (EV1-E8/E9 5'-AAGAGTGAGCCATGCTGTACACCA-3') or were located downstream the stop codon for the three other genes (TK1-E7 5'-GCTACAGCAGAGGCGTGGACCCT-3'; SYNGR2-E4 5'-CGTCTCCAAACAGCATCCCA-3'; and EVIN2-E15 5'-GAGGCCTGAAGGGGCCCCAGGGAG-3') genes. The GAPDH probe was amplified with two primers specific G3PDH-F ²and G3PDH-R as described.
After agarose gel electrophoresis, PCR products were visualized by ethidium bromide staining (see figure 3b). Two main cDNAs with sizes corresponding to 2.6 kb and 1.5 kb were detected in lymphoblastoïd cells or in the skin. The structure of *Evin1* transcripts is shown in Figure 3b. Exons 2 to 20 are contained in the 2.6 kb cDNA species while exons 11 to 18 are skipped in the 1.5 kb cDNA species.
Amplification products were excised and purified using QIAquick spin columns (Qiagen) before labeling with ³²P-dCTP using Ready-To-Go DNA labeling kit (Amersham Pharmacia Biotech). EVIN1 cDNAs were also obtained using primer EV1-ATG and a primer located downstream the stop codon (EV1-E20 5'-CCAGCTCCAGCCTAGGCGCAGCTG-3').
EVIN1 and EVIN2 cDNAs were cloned into plasmid pCR3.1, using the Eukaryotic TA cloning kit (Invitrogen), and sequenced.
The full-length cDNA sequence of the Evin-1 gene is shown in SEQ ID NO:1. The full-length cDNA sequence of the Evini-2 gene is shown in SEQ ID NO:2.
The predicted sequences of the putative proteins encoded by Evin-1 and 2 genes (respectively SEQ ID NO:5 and SEQ ID NO:6) were analyzed using web-based European molecular biology open software suite (EMBOSS) (ftp://ftp.uk.embnet.org/pub/EMBOSS). More specifically, the amino acid sequence of the putative proteins encoded by the Evin-1 2.6 kb and 1.5 kb mRNAs is shown in Figure 5. Analysis of the sequence revealed eight putative transmembrane domains (shaded) and two leucine zipper motifs (underlined) predicted in the 805 amino acid EVIN-1 protein. The position of the mutated amino acid in Evin-1 is also shown in Figure 5.
Fig. 7 shows an alignment of amino acid sequences of the putative proteins encoded by the Evin-1 and Evin-2 genes. Transmembrane domains (shaded) and leucine zipper motifs (double underlined) predicted in the two proteins are shown. Conserved amino acid are indicated (*). Spots correspond to amino acids conserved between the Evin-1 protein and putative mouse protein BC004840. The amino acids found mutated in Evin-1 (R94 and E576) or Evin-2 (E377) gene are boxed.

### BIBLIOGRAPHY

Jablonska *et al*., Epidermodysplasia verruciformis as a model in studies on the role of papovaviruses in oncogenesis. *Cancer Res.,* 1972; 32 :583-589

McKusick, VA : Mendelian Inheritance in Man. A Catalog of Human Genes and Genetic Disorders. The Johns Hopkins University Press. Baltimore, London 1998

Orth G. *et al.* Characterization of two types of human papillomaviruses in lesions of epidermodysplasia verruciformis. *Proc Natl Acad Sci USA* 75: 1537-1541, 1978

Orth G. *et al.* Characteristics of the lesions and risk of malignant conversion as related to the type of the human papillomavirus involved in epidermodysplasia verruciformis, *Cancer Res.* 39, 1074-1082, 1979

Orth, G. *et al.* Epidermodysplasia verruciformis, In: Howley PM, Salzman NP (eds). The Papillomaviruses, Vol. 2, New York: Plenum Press, 1987, pp. 199-243

Boxman ILA. *et al.* Detection of human papillomavirus DNA in plucked hairs from renal transplant recipients and healthy volunteers. *J. Invest. Dermatol* 108 : 712-715, 1997

Astori G. *et al.* Human papillomaviruses are commonly found in normal skin of immunocompetent hosts. *J. Invest dermatol* 110 :752-755, 1998

Rueda LA and Rodriguez G. Verrugas humanas por virus papova. Correlacion clinica. Histologica y ultraestructural. Med. *Cutanea Ibero Lat Am* 2: 113-136,1976

Majewski S. *et al.* Epidermodysplasia verruciformis. Immunological and nonimmmunological surveillance mechanisms: role in tumor progression. *Clin. Dermatol.* 15 :321-334, 1997

Plummer S.J., *et al.* Mapping of 228 ESTs and 26 genes into an integrated physical and genetic map of human chromosome 17. *Genomics* 45: 140-146, 1997

Kelsell D.P. *et al.,* Close mapping of the focal non-epidermolytic palmoplantar keratoderma (PPK) locus associated with oesophageal cancer (TOC). *Hum Mol Genet* 5: 857-860, 1996

Nair R .P. *et al.* Evidence for two psoriasis susceptibility loci (HLA and 17q) and two novel candidate regions (16q and 20p) by genome-wide scan. *Hum Mol Genet* 6: 1349-1356, 1997

Quinn AG *et al.,* Basal cell carcinomas and squamous cell carcinomas of human skin show distinct patterns of chromatosome loss. *Cancer Res* 54 : 4756-4759, 1994

Rehman I. *et al.,* Genetic change in actinic keratoses. *Oncogene* 12: 2483-2490, 1996

Tomfohrde J. *et al.,* Gene for familial psoriasis susceptibility mapped to the distal end of human chromosome 17q. *Science* 264: 1141-1145, 1994

Favre M. *et al.* Psoriasis: a possible reservoir for human papillomavirus type 5 , the virus associated with skin carcinomas of epidermodysplasia verruciformis. *J. Invest Dermatol* 110 :311-317, 1998

Majewski S. *et al.,* Is human papillomavirus type 5 the putative autoantigen involved in psoriasis ? *J. Invest Dermatol* 111: 541-542, 1998

Pfister H. and ter Schegget J. , Role of HPV in cutaneous premalignant and malignant tumors. *Clin Dermatol* 15:335-348, 1997

Ramoz *et al.* A Susceptibility Locus for Epidermodysplasia Verruciformis ? an Abnormal Susceptibility to Infection with the Oncotgenic Human Papillomavirus Type 5, Maps to Chromosome 17qter in a Region Containing a Psoriasis Locus, *The Journal of Investigative Dermatology,* vol. 112, n° 3, pp. 259-263, 1999

Ramoz *et al*. Evidence for a Nonallelic Heterogeneity of Epidermodysplasia Verruciformis with Two Susceptibility Loci Mapped to Chromosome Regions 2p21-p24 and 17q25, vol. 114, n° 6, pp. 1148-1153, June 2000,

Ramoz N, Thèse universitaire-Université Paris 7-Denis Diderot, soutenue le 21 Juin 2000 : Identification de loci contenant des gènes de prédisposition ou de résistance à l'infection par les papillomavirus humains oncogènes : le modèle de l'épidermydodysplasie verruciforme.

Büssow, K., Cahill, D., Nietfeld, W., Bancroft, D., Scherzinger, E., Lehrach, H. & Walter, G. (1998). A method for global protein expression and antibody screening on high-density filters of an arrayed cDNA library. *Nucleic. Acids Res.* **26,** 5007-5008.

Emili, A. Q. & Cagney, G. (2000). Large-scale functional analysis using peptide or protein arrays. *Nat. Biotechnol.* **18,** 393-397.

MacBeath, G. & Schreiber, S. L. (2000). Printing proteins as microarrays for high-throughout function determination. *Science* **289,** 1760-1763.

Zhu, H., Klemic, J.F., Chang, S., Bertone, P., Casamayor, A., Klemic, K.G., Smith, D., Gerstein, M., Reed, M.A. & Snyder, M. (2000). Analysis of yeast protein kinases using protein chips. *Nat. Genet.* **26,** 283-289.

Holt, L. J., Büssow, K., Walter, G. & Tomlinson, M. (2000). By-passing selection: direct screening for antibody-antigen interactions using protein arrays. *Nuclic Acids Res.* **28,** e72.

Mahlknecht, U., Ottmann, O. G. & Hoelzer, D. (2001). Far-Western based protein-protein interaction screening of high-density protein filter arrays. *J. Biotechnol.* **88,** 89-94.

Vidal, M. & Legrain, P. (1999). Yeast forward and reverse "n"-hybrid systems. *Nucleic Acids Res.* **27,** 919-929.

Joung, J. K., Ramm, E. I. & Pabo, C. O. (2000). A bacterial two-hybrid selection system for studying protein-DNA and protein-protein interactions. *Proc. Natl. Acad. Sci. USA* **97,** 7382-7387.

Walhout, A. J. M., Sordella R., Lu, X., Hartley J. L., Temple, G. F., Brasch, M. A., Thierry-Mieg, N., Vidal, M. (2000). Protein interaction mapping in *C. elegans* using proteins involved in vulval development. *Science* **287,** 116-122.

Uetz, P., Giot, L., Cagney, G., Mansfield, T. A., Judson, R. S., Knight, J. R., Lockshon, D., Narayan, V. (2000). A comprehensive analysis of protein-protein interactions in *Saccharomyces cerevisiae. Nature* **403,** 623-627.

Rebar, E. J. & Pabo. C. O. (1994). Zinc finger phage: affinity selection of fingers with new DNA-binding specificities. *Science* **263,** 671-673.

## Claims

1. A purified nucleic acid, **characterized in that** it hybridizes to a nucleotide sequence selected among SEQ ID NO:1, NO:2, NO:3 and NO:4, under stringent conditions.

2. The purified nucleic acid according to claim 1, **characterized in that** it is at least 15 nucleotides long.

3. The purified nucleic acid according to Claim 1 or 2, **characterized in that** it is selected among
a) Evin-1 cDNA as shown in SEQ ID NO:1 or Evin-1 gene as shown in SEQ ID NO:3;
b) Evin-2 cDNA as shown in SEQ ID NO:2 or Evin-2 gene as shown in SEQ ID NO:4
c) a fragment of a nucleic acid selected among SEQ ID NO:1, NO:2, NO:3 and NO:4; and
d) a mutated fragment of a nucleic acid selected among SEQ ID NO:1, NO:2, NO:3 and NO:4, said mutated fragment having a punctual mutations with respect to the corresponding wild type fragment.

4. The purified nucleic acid according to any of Claims 1 to 3, **characterized in that** it consists of a mutated fragment from SEQ ID NO:1, having one of the following punctual mutations: C280T, G1726T, wherein 280 or 1726 refer to the position of the mutated nucleotide in SEQ ID NO:1.

5. The purified nucleic acid of Claim 4, **characterized in that** it is selected among SEQ ID NO:7 or SEQ ID NO: 8 or fragments thereof.

6. The purified nucleic acid of Claim 3, **characterized in that** it consists of a mutated fragment of SEQ ID NO:2, having at least the following punctual mutation: G1129T, wherein 1129 correspond to the position of the mutated nucleotide in SEQ ID NO:2.

7. The purified nucleic acid of Claim 6, **characterized in that** it consists of SEQ ID NO:9 or a fragment thereof.

8. The purified nucleic acid according to any of Claims 1 to 7 **characterized in that** it has length comprised between 100 to 250 pb.

9. The purified nucleic acid according to any of Claims 1 to 8, **characterized in that** it encodes a polypeptide involved in the susceptibility for developing Epidermodysplasia verruciformis.

10. The purified nucleic acid of Claim 9, **characterized in that** it comprises a mutated gene whose expression is correlated to an increased susceptibility to infection to Human Papilloma Virus (HPV), to Epidermodysplasia verruciformis, and/or to skin diseases such as psoriasis.

11. The purified nucleic acid of Claim 10, **characterized in that** said mutated gene is the Evin-1 gene having one of the following mutations: C280T (changing an arginine codon into a stop codon) and G1726T (changing a glutamic acid codon into a stop codon).

12. The purified nucleic acid of Claim 11, **characterized in that** said mutated gene is the Evin-2 gene having the mutation G1129T (changing a glutamic acid codon into a stop codon).

13. A purified polypeptide **characterized in that** it is encoded by the nucleic acid according to anyone of Claims 9-12.

14. The purified polypeptide of Claim 13, **characterized in that** it consists of the Evin-1 protein as shown in SEQ ID NO:5.

15. The purified polypeptide of Claim 13, **characterized in that** it consists of the Evin-2 protein as shown in SEQ ID NO:6.

16. The purified polypeptide of Claim 13, **characterized in that** it consists of a truncated form of SEQ ID NO:5 at amino acid positions 94 or 576 or of a truncated form of SEQ ID NO:6 at amino acid position 377, the expression of said truncated form being involved in an increased susceptibility to infection to Human Papilloma Virus (HPV), to Epidermodysplasia verruciformis, and/or to skin diseases such as psoriasis in human.

17. An antibody **characterized in that** it specifically recognizes the polypeptide according to any of Claims 13 to 16.

18. The antibody according to Claim 17, **characterized in that** it is directed against the C-terminal part of SEQ ID NO:5 starting from amino acid position 94 or from amino acid position 576.

19. The antibody according to Claim 18, **characterized in that** it is directed against the C-terminal part of SEQ ID NO:6 starting from amino acid position 377.

20. A recombinant vector **characterized in that** it comprises a nucleic acid according to anyone of Claims 1 to 12.

21. The recombinant vector according to Claim 20, **characterized in that** it is a plasmid comprising a nucleic acid according to any of Claims 9 to 12, said nucleic acid encoding a polypeptide involved in the susceptibility for developing Epidermodysplasia verruciformis and said plasmid further comprising appropriate sequences for expression of said polypeptide in a host cell.

22. A host cell **characterized in that** it is transformed by a vector according to Claim 20 or Claim 21.

23. The host cell according to Claim 22, **characterized in that** it is a bacteria, preferably, an *E. coli* cell.

24. The host cell according to Claim 23, **characterized in that** it is the bacterial strain deposited at the C.N.C.M on the 18^{th} of July 2001, under accession number 1-2704.

25. The host cell according to Claim 22, **characterized in that** it is an eukaryotic cell, especially a mammalian cell, and more preferably a human cell.

26. A pharmaceutical composition comprising a nucleic acid according to any of Claims 1 to 12 and a pharmaceutically acceptable vehicle.

27. A method of screening of molecules having a therapeutic activity against human papillomavirus infection, Epidermodysplasia verruciformis, and/or psoriasis, said method comprising:
a) culturing eukaryotic cells transformed by the nucleic acid according to Claims 3, 11 or 12;
b) contacting said host cells with molecules susceptible to have a therapeutic activity against human papillomavirus infection, Epidermodysplasia verruciformis, and/or psoriasis; and
c) selecting the molecule(s) which restore(s) the expression of a wild type gene.

28. The method according to claim 27, wherein the selected molecules in step c) are those which restore the transcription and/or the translation of the wild type gene.

29. A method for the identification of an allelic form of Evin-1 or Evin-2 gene comprising:
a) isolating a nucleic acid which hybridizes to any of SEQ ID NOs :1-4 under stringent conditions from a biological sample of a patient having an increased susceptibility to infection to HPV, or to Epidermodysplasia verruciformis;
b) sequencing the purified nucleic acid; and
c) detecting a mutation by comparison of the novel sequenced nucleic acid with the corresponding wild-type sequence, said mutation being indicative of an allelic form of Evin-1 or Evin-2 gene.

30. A method for detecting *in vitro* a susceptibility to infection to HPV, to Epidermodysplasia Verruciformis, and/or to a skin disease such as psoriasis, said method comprising:
a) extracting genomic DNA or RNA from a biological sample; and,
b) detecting in either Evin-1 or Evin-2 gene, specific mutations involved in an increased susceptibility to Epidermodysplasia verruciformis, to infection to HPV and/or to skin diseases such as psoriasis.

31. A method for detecting *in vitro* a susceptibility to Epidermodysplasia verruciformis, to infection to HPV and/or to a skin disease such as psoriasis, in a human biological sample, said method comprising:
a) extracting proteins from a biological sample; and,
b) detecting truncated forms of either Evin-1 or Evin-2 protein involved in an increased susceptibility to Epidermodysplasia verruciformis, to infection to HPV and/or to a skin disease such as psoriasis.

32. A kit for detecting *in vitro* susceptibility to Epidermodysplasia verruciformis, to infection to HPV and/or to a skin disease such as psoriasis, said kits comprising at least one of the following components:
a) an purified nucleic acid according to any of Claims 1 to 12 or a vector according to Claim 20 or 21;
b) optionally, appropriate buffers for DNA or RNA extraction and/or DNA or RNA hybridization reaction, and/or for detection of the mutated DNA or RNA.

33. A kit for detecting *in vitro* susceptibility to Epidermodysplasia verruciformis, to infection to HPV and/or to skin diseases such as psoriasis, in a human biological sample, said kits comprising the following components:
a) antibodies according to any of Claims 17 to 19;
b) optionally, appropriate buffers for protein extraction and/or antigen-antibody immunological reaction, and/or for detection of the antigen-antibody immunological reaction.

34. A DNA chip **characterized in that** it contains at least a nucleic acid according to any of Claims 1 to 12.

35. A protein chip **characterized in that** it contains at least an antibody according to any of Claims 17 to 19, or a polypeptide according to any of Claims 13 to 16.

36. Use of a nucleic acid according to any of Claims 1 to 12 for *in vitro* detection of a susceptibility to human papillomavirus infection, to a skin disease such as psoriasis and/or to Epidermodysplasia verruciformis.

37. Use of a nucleic acid according to any of Claims 1 to 12 for the screening of proteins involved in the development of psoriasis, HPV-associated disease or Epidermodysplasis verruciformis.

38. Use of a nucleic acid according to any of Claims 1 to 12 for the preparation of pharmaceutical composition appropriate for treating or preventing human papillomavirus infection, psoriasis and/or Epidermodysplasia verruciformis.

39. A bacterial host cell containing one of the nucleic acids according to claims 1 to 12 and 20 to 21.

40. A process for the production of a nucleic acid according to any of Claims 1 to 12 comprising:
a) culturing the cell according to any of claims 22 to 25 or the bacterial host cell according to claim 39;
b) separating the nucleic acid according to any of claims 1 to 12 from the cellular components.

41. A process for the production of the protein corresponding to the Evin-1 gene or Evin-2 gene **characterized by** the following step:
a) culturing a host cell according to any of claims 22 to 25 in conditions appropriate for expression of the protein corresponding to Evin-1 or Evin-2 gene; and,
b) purifying the protein according to any of claims 13 to 16 from the cellular components.


SEQUENCE LISTING

<110> INSTITUT PASTEUR
      INSERM

<120> NUCLEIC ACIDS AND POLYPEPTIDES INVOLVED IN THE
      PREDISPOSITION TO EPIDERMODYSPLASIA VERRUCIFORMIS, TO
      PSORIASIS AND/OR TO INFECTION TO HUMAN PAPILLOMA VIRUS

<130> 4958 -DBO

<140> unknown
<141> 2001-07-24

<160> 31

<170> PatentIn Ver. 2.1

<210> 1
<211> 2418
<212> DNA
<213> human

<220>
<221> misc_feature
<222> Complement((1)..(2418))
<223> Evin-1 cDNA

<400> 1
atggcccagc cactggcctt catcctcgat gtccctgaga ccccagggga ccagggccag 60
ggccccagcc cctatgatga aagcgaagtg cacgactcct tccagcagct catccaggag 120
cagagccagt gcacggccca ggaggggctg gagctgcagc agagagagcg ggaggtgaca 180
ggaagtagcc agcagacact ctggcggccc gagggcaccc agagcacggc cacactccgc 240
atcctggcca gcatgcccag ccgcaccatt ggccgcagcc gaggtgccat catctcccag 300
tactacaacc gcacggtgca gcttcggtgc aggagcagcc ggcccctgct cgggaacttt 360
gtccgctccg cccggcccag cctccgcctg tacgacctgg agctggaccc cacggccctg 420
gaggaggagg agaagcagag cctcctggtg aaggagctcc agagcctggc agtggcacag 480
cgggaccaca tgcttcgcgg gatgccctta agcctggctg agaaacgcag cctgcgagag 540
aagagcagga ccccgagggg gaagtggagg ggccagccgg gcagcggcgg ggtctgctcc 600
tgctgtggcc ggctcagata tgcctgcgtg ctggccttgc acagcctggg cctggcgctg 660
ctctccgccc tgcaggccct gatgccgtgg cgctacgccc tgaagcgcat cgggggccag 720
ttcggctcca gcgtgctctc ctacttcctc tttctcaaga ccctgctggc tttcaatgcc 780
ctcctgctgc tgctgctggt ggccttcatc atgggccctc aggtcgcctt cccacccgcc 840
ctgccgggcc ctgcccccgt ctgcacaggc ctggagctcc tcacaggcgc gggttgcttc 900
acccacaccg tcatgtacta cggccactac agtaacgcca cgctgaacca gccgtgtggc 960
agccccctgg atggcagcca gtgcacaccc agggtgggtg gcctgcccta caacatgccc 1020
ctggcctacc tctccactgt gggcgtgagc ttctttatca cctgcatcac cctggtgtac 1080
agcatggctc actctttcgg ggagagctac cgggtgggca gcacctctgg catccacgcc 1140

atcaccgtct tctgctcctg ggactacaag gtgacgcaga agcgggcctc ccgcctccag 1200
caggacaata ttcgcacccg gctgaaggag ctgctggccg agtggcagct gcggcacagc 1260
cccaggagcg tgtgcgggag gctgcggcag gcggctgtgc tggggcttgt gtggctgctg 1320
tgtctgggga ccgcgctggg ctgcgccgtg gccgtccacg tcttctcgga gttcatgatc 1380
cagagtccag aggctgctgg ccaggaggct gtgctgctgg tcctgcccct ggtggttggc 1440
ctcctcaacc tgggggcccc ctacctgtgc cgtgtcctgg ccgccctgga gccgcatgac 1500
tccccggtac tggaggtgta cgtggccatc tgcaggaacc tcatcctcaa gctggccatc 1560
ctggggacac tgtgctacca ctggctgggc cgcagggtgg gcgtcctgca gggccagtgc 1620
tgggaggatt ttgtgggcca ggagctgtac cggttcctgg tgatggactt cgtcctcatg 1680
ttgctggaca cgctttttgg ggaactggtg tggaggatta tctccgagaa gaagctgaag 1740
aggaggcgga agccggagtt tgacattgcc cggaatgtcc tggagctgat ttatgggcag 1800
actctgacct ggctgggggt gctcttctcg cccctcctcc ccgccgtgca gatcatcaag 1860
ctgctgctcg tcttctatgt caagaagacc agccttctgg ccaactgcca ggcgccgcgc 1920
cggccctggc tggcctcaca catgagcact gtcttcctca cgctgctctg cttccccgcc 1980
ttcctgggcg ccgctgtctt cctctgctac gccgtctggc aggtgaagcc ctcgagcacc 2040
tgcggcccct tccggaccct ggacaccatg tacgaggccg gcagggtgtg ggtgcgccac 2100
ctggaggcgg caggccccag ggtctcctgg ctgccctggg tgcaccggta cctgatggaa 2160
aacaccttct ttgtcttcct ggtgtcagcc ctgctgctgg ccgtgatcta cctcaacatc 2220
caggtggtgc ggggccagcg caaggtcatc tgcctgctca aggagcagat cagcaatgag 2280
ggtgaggaca aaatcttctt aatcaacaag cttcactcca tctacgagag gaaggagagg 2340
gaggagagga gcagggttgg gacaaccgag gaggctgcgg caccccctgc cctgctcaca 2400
gatgaacagg atgcctag 2418

<210> 2
<211> 2389
<212> DNA
<213> human

<220>
<221> misc_feature
<222> Complement((1)..(2389))
<223> Evin-2 cDNA

<400> 2
atgctgctgc cgcggtcggt gtcatcggag cgggcccctg gggtgccgga gccggaggag 60
ctgtgggagg cagagatgga gcggctgcgc ggctctggga cgcccgtgcg cgggctgccc 120
tatgccatga tggacaagcg cctcatctgg cagctgcggg agcccgcggg ggtgcagacc 180
ttgcgctggc agcggtggca gcgccggcgg cagacggtgg aaaggcgcct gcgggaggca 240
gcgcagcggc tggcccgggg ccttgggctc tgggaggggg cgctctacga gatcgggggc 300
ctcttcggca caggaattcg gtcctacttc accttcctcc gcttcctgct gctactcaac 360
ctgctgagcc tgctgctcac cgcaagcttc gtgctgctgc ccctggtctg gctccgcccc 420
cctgacccag gccccaccct gaacttgacc ctccagtgcc ctggtagccg ccagtccccg 480
cctggcgttt tgaggttcca caatcaactt tggcatgttt tgactggcag ggccttcacc 540
aacacctatc tcttctacgg tgcgtaccga gtggggccgg agagcagctc cgtgtacagc 600
atccgcctgg cctacctcct cagcccgctg gcctgcctgc tcctctgctt ctgtgggact 660
ctgcggcgga tggtgaaggg gctgccgcag aagactctgc tgggtcaggg ctatcaggcg 720
cctctcagcg ccaaggtctt ctcctcatgg gacttctgca tccgggtgca ggaagcagcc 780

accatcaaga agcatgagat cagcaacgag ttcaaggtgg agctggagga gggccgtcgc 840
ttccagctga tgcagcagca gacccgggcc cagacggcct gccgcctgct ctcctacctg 900
cgggtcaacg tactcaacgg gctcctggtg gttgggccca tcagcgccat cttctgggct 960
accaagtact cacaggacaa caaggaggtg tcaggcaact gcattcattt aatcctggcc 1020
agaactgcgg gggagtccct gtttctgctg ctccagtacc tgccccctgg ggtcatcgcc 1080
ctggtcaact tcctgggtcc cctgctgttc acatttctgg tccagctgga gaactaccct 1140
cccaacacgg aggtcaacct cactctgatc tggtgcgtgg tgctgaagct ggccagcttg 1200
gggatgttct ccgtctccct gggtcagacc atactgtgca ttggcagaga caagagcagc 1260
tgtgagtcct acggctacaa cgtttgtgac tatcagtgct gggagaactc cgtgggggag 1320
gagctgtaca agctgagtat cttcaacttc ctcctcaccg tggccttcgc cttcctggtc 1380
accctgcctc ggaggctgct ggtggaccgg ttctcaggcc ggttctgggc ctggctggaa 1440
cgggaggagt tcctggtccc caagaatgtg ctggacatcg tggcggggca gacggtcacc 1500
tggatgggcc tcttctactg ccccctgctg cccctgctga atagcgtctt cctcttcctc 1560
accttctaca tcaagaaggt gacggctcat ggctgggggt acaccctcct gaagaactcc 1620
agggcatctt cgcggccctt ccgtgcctcc agctccacct tcttcttcca gctagtgctc 1680
ctcctgggcc tgcttctggc tgcagtgccc ctgggctatg tggtcagcag ccccacttgg 1740
ccatctctcg ccagcatcca ctcctcctgg gactgcggcc tcttcaccaa ctactcagca 1800
ccctggcaag tggtcccgga gctggtggcc cttgggctcc cgcccattgg ccagcgtgcc 1860
ctccactacc tgggctccca cgccttcagc ttccccctcc tcatcatgct cagccttgtc 1920
ctgacggtgt gcgtctccca gacccaggcc aatgccaggg ccatccacag gctccggaag 1980
cagctggtgt ggcaggttca ggagaagtgg cacctggtgg aggacctgtc gcgactgctg 2040
ccggagccag gcccgagcga ctctccgggc cccaagtacc ctgcctccca agcttcgcgc 2100
ccgcagtcct tctgccccgg atgcccatgc cctggctccc cgggccacca ggccccgcgg 2160
ccgggcccct ccgtcgtgga tgccgcggga ctgcgttccc cttgccctgg acagcacggt 2220
gccccggcct ccgcccgcag attccgcttc cccagcggcg cggagctgta acccctaccc 2280
ctgcctcccc gaagcctccc tggggcccct tcaggcctcc ttactccatc ttccagaccc 2340
ctggcgacca ccgcccctct cagtggctcc agggcctccc tcagaggtc 2389

<210> 3
<211> 16098
<212> DNA
<213> human

<220>
<221> misc_feature
<222> (1)..(16098)
<223> Evin-1 gene

<400> 3
tgcggctact ccccgggccc cggcagacct gcgactggcc cagtcgcccc ctggggccct 60
ttgccactcc cttggcaagg agagccgaga cctcagttcc cggcggctct tgcggggcac 120
aggtgagccc tggctgcgcg cgcggcccct cctccccggc gcctcccagg tgagcggccg 180
cgatcccggt cccgggtccc gccagcccca gctgcttctc ctatgcgggg acagcggcaa 240
tccccccctga attcctttcg gcctctgggg ccatttggca gccgagttcc ctccccgggt 300
gctcctggag ctcccagggc ttgaccctcc ggctcggaca gagctgggag cggcaggggc 360
ggggaggaca gtgcggggag caaaatccgc agcccccacc caggtccccc agccgagcgc 420
cggggcacgg ggaggggagg ggagggggtg tggccgggat gggaaggggc gggggacagg 480

gaggccggct ggacgggttc ggtggcccca gagttgcagc cgctctgggc tctgggccca 540
ggttgcggtt ctccctgggt agcggctgct cccgggaagc tcgctgccgg ctggggtggg 600
ggttcctgga aacgggcgcc acccagcctc tccccaccta gctcaggaat ggtgcgcctg 660
gaaactccgc tggacttgcc tgggcctcgg ggcggagtgc tggggcgctg ggtgcccgag 720
tccccgggtt ccctgagctt agaagggcct gtctggagga agggattcca gctctaggga 780
ctgggtgggg cgtcctcagc ccctctttcg gcagctgtcc cgtgttaggc tgggtggggc 840
cgcctcggac ccagctctgg gctggttttc ccacgtcttt gctcctgctg gagggagggg 900
gtggtgtggg gcctgggccc gaagatggca cccaggacac tgagggaggg cgccgtccgg 960
tcctgtgact ggcacccctg caaggagctg ctctgtcggg gcgcccccgg gctttgcctg 1020
tccgaggaaa gcgcccttca acccggccga ctttgagccc tgagttcctg agggtgagcg 1080
caggaggagt ccagggcaga ggccggcagg actcgctggt ggctgggagc tgagtcctgg 1140
ctccactctc acagatgaac gggccccgag cccagggccc cagacaaggc tgctctggtt 1200
ccagttgtgc cgggtgacgg cagagccagg acaaaggagg cctgttgagg tcctggccac 1260
ccccagccgg ccgccctggg atgatggaat gcagggcagt gggtcttctg agaacatgag 1320
gaccttggcg ccctgacggc ggtggcatgg agtgacctct gtgtgtgggg gaggagcccc 1380
ctgtgctgct tccgcctggg gctggttggg ggtgagggct ccagcagcta atttcctgct 1440
agaggaagtt ctttgaggtc acagggcaga cgcagagaag gcctacagcc tactctgacc 1500
tttctctcca aaaaggaggt gtctctgtgc cagaaccctg ggcatggaga tcctgaaagg 1560
ggctgtgtct acaggcaccc tacctcttcc ccatttccca gggactggcg acatcgagga 1620
aacagatgtg gaactgcaga ccccacaccc ttccttcctc tctcccctcc cctgacactg 1680
gagggggccc cccattccat tctggcatga ggcttattcg gtgcctcacc atttatgtac 1740
ctggggtgat aggggggtgg gcgggactgt ctgggctgtg agtggcacct cctgtggttc 1800
ccccaggaat gggtggtgtg gacagctgcc aggcgttgaa gaccacccct ggctctgtgc 1860
ccctgtctca tcagatgggg gctccggagg tggcgcccag gctctgagct accctaggtc 1920
tgcagactag cgggcattgg ccagagacat ggcccagcca ctggccttca tcctcgatgt 1980
ccctgagacc ccaggggacc aggggtaagc gctctggggc tgggggctgg aggctggaat 2040
gttccacccc ccacggagag gtcacctgag caggggctgc tggaaggaga gaggtggctg 2100
gagcctgacc cccccgccaa ccccctctgc agccagggcc ccagccccta tgatgaaagc 2160
gaagtgcacg actccttcca gcagctcatc caggagcaga gccagtgcac ggcccaggag 2220
gggctggagc tgcagcagag agagcgggag gtgacaggtg agctcaggcc tggatgctgg 2280
gtggtgtgga ccaagacttg ggtgccccag cccctcagga cgggcctccc agctctgcag 2340
gcctgggatg gggtgggaga tactgtggcc aatggcatct ccaggacccc tggtgagtct 2400
ttcttgcttt gtaggaagta gccagcagac actctggcgg cccgagggca cccagagcac 2460
ggccacactc cgcatcctgg ccagcatgcc cagccgcacc attggtgagt gggaccctca 2520
gcctcggcct cggccccggc cccgagttga ccccagtccc tggggctggg ctgggcttcc 2580
tgccaggcat ctccctgggt agtggctgct cccgtagctc agagccaggc ccagggcggg 2640
ctcctagccc atcccatctg ctgcctgggg gcccagcaca gccccatgtg gccctcgctg 2700
ggctccaaaa atgactccag agtcatcacg gggtaaaaat aggctttcgg aatgcagtgg 2760
tgcctgaagt ctttgttgaa agggttttcc tgggtcctgg gaaggggtga gggacgcaga 2820
ggcctgtggt gagcccatcc tgaggggagg gaggcctggc cctgcccggc ccggccctgc 2880
ccagctgcct ccataggccg cagccgaggt gccatcatct cccagtacta caaccgcacg 2940
gtgcagcttc ggtgcaggag cagccggccc ctgctcggga actttgtccg ctccgcccgg 3000
cccagcctcc gcctgtacga cctggagctg gaccccacgg ccctggagga ggagggtgag 3060
tgagtgggca ctggagcccc agtggcggac accggcctgg ggtggggtgc aagaggccag 3120
cctggcctgg gcggtggctg gggtgctgca gagccagagg cccctctcct atcccttagc 3180
aggctgcctg aaggcccccc cgtccctctg cagggaccca cggggacagg gaagtggggg 3240
tcttacctca caagcgcctt ggagtgcttg gagggctgtg gccctgggcc ccgagtcggg 3300
gaccgtgcgt gggaaacagg cccctgtgct ctgtgagctt cctgcttgtc gctgactgaa 3360

gcaaacacac ggctgattgg cgactgaagc tcaaataagg gcaccgaggt gtctcgggac 3420
ggtgccgcag ggacacacag gcctgaccag ggtcccggct tcgggctgca gggaggggca 3480
cttggggatg ggcaggacct ccctctccct ctcgcagaga agcagagcct cctggtgaag 3540
gagctccaga gcctggcagt ggcacagcgg gaccacatgc ttcgcgggat gcccttaagc 3600
ctggctgaga aacgcagcct gcggtgagca gccccgaccc tgaccatgcc gccgctgctg 3660
cgcccccaga cccacttcct cagctgggct aggtggtggg ggagaagggg ctgagcccgt 3720
gggtggccgg tctccaggaa ggagagagtc aggcccctca ttggtccagg cacctatggc 3780
tgcctctgtc cccagagaga agagcaggac cccgaggggg aagtggaggg gccagccggg 3840
cagcggcggg gtctgctcct gctgtggccg gctcagatat gcctgcgtgc tggtatgggc 3900
ccctggtctg ccctggggct ggggcggctg ggcagggctg gggtggcctc agtgcctcct 3960
ccggcctcac cctcagggcc tctctgcctg tgcccgcagg ccttgcacag cctgggcctg 4020
gcgctgctct ccgccctgca ggccctgatg ccgtggcgct acgccctgaa gcgcatcggg 4080
ggccagttcg gctccagcgt gctctcctac ttcctctttc tcaagaccct gctggctttc 4140
aatgccctcc tgctgctgct gctggtggcc ttcatcatgg gccctcaggt cgccttccca 4200
cccgccctgc cgggccctgc ccccgtctgc acaggcctgg agctcctcac aggcgcggtg 4260
aggaagccct aggactgggg gacggggggt gcctgtcttc tgctaccctg gtaccgtgtt 4320
cccttcttgt cccagctccc ctggggcccc caacccttgc cccgctgtga ctccaggtgg 4380
ccacagccag ctccccgcct ccccctcaat gaccctggat gctcatcgtg ctggggttcc 4440
catggggctg gaggcccaag attgctgtgg ccaaggcccc tccctgtttc cctcactgcc 4500
cctgcctggc ctcctcctgc tctgcccctg tggctgtcag gcctgagggt cgggtggggc 4560
gtggggaaag tccctgactc ggctggatct ccctgtggca gggttgcttc acccacaccg 4620
tcatgtacta cggccactac agtaacgcca cgctgaacca gccgtgtggc agccccctgg 4680
atggcagcca gtgcacaccc agggtgggtg gcctgcccta caacatgccc ctggcctacc 4740
tctccactgt gggcgtgagc ttctttatca cctgcatcac cctggtgtac aggtaacgcc 4800
tccacctcat gacccaggct ccgagctcca ctcatccatc cattcatctt tcgacccatt 4860
cattgattct tccctcttcc tttcttcctt ctgtccatct acctgtctgc tctcatccac 4920
tcacctatcc aactatccac ttacccaccc atctacccat ccatctacct acccacccat 4980
ccacccattc atccatccac ccacccatcc acccactcaa ttcacccacc catttaccca 5040
ttcatccatc cacctaccta tctacccact caattcaccc ccccatccac ccactcaatt 5100
cacccaccca tccatctacc cacccattca cccaatcagt tcacccaccc atccacccat 5160
ccacccattc atccattcac ccacccatcc atccattcac acattcaccc attcacccat 5220
ccacccaccc atccacccat tcatccatct acccacccat ccatccactc agtttaaccc 5280
acccatccac ccattcatcc atccacccac tcattcatcc acgcatccat ccattcgcac 5340
atccatccac ctccttatct atccacccat tcatccaccc atccgttcat ccatccaccc 5400
atccacccac acatttattc atccatccat ccacccatcc acccatgcat ctacgtattc 5460
cacaaacatt ctctgtgctc agctcagggc aggatggtaa atgtcctagg ggggcttgct 5520
gtctctggga gaagatggac cactgaatgg gctgggacaa tccatgaggc ttagtggtgt 5580
ttccccagtg ttatgggagc actggggaag gagtcaggct gcttcccagg ggaggcggca 5640
cttgccctga gaggggaagg ttagataaga gtggggatgg ataaggaagc agggaagatg 5700
ttttggtcat tgggaccagc agtgcaaagg ccctgaggtg agcccagcat cttctggtcc 5760
atgtctcgct gggaggcagc cagccaagtc cgcggtgaat gcagccctgc cccctcttct 5820
aggcagatgt ggctggtggg tgctatgttg gatgagactg gtgtagaggg ctggtggtgg 5880
ggcaggggtg tggcaggagg cccagtcagg gcccagatgg ctcctgagtg ggtggtgggg 5940
tctggctgcc cagagcatcc ggtctgcctt ctccgcttgg cctctgcccg tcagcttgtt 6000
ggttgccatg tctgtgtctg cttctccgca gcatggctca ctctttcggg gagagctacc 6060
gggtgggcag cacctctggc atccacgcca tcaccgtctt ctgctcctgg gactacaagg 6120
tgacgcagaa gcgggcctcc cgcctccagc aggacaatat tcgcacccgg ctgaaggtga 6180
gtggccccgg agcaccaagc tggcctgccc agctccctgc aggcctggcc ctgggtccac 6240

ttaggaccat gtgaccagaa ccatggtcta gagggagggc cagctgtccg tgagctgaac 6300
tcggggcgct gggcaggcgt ggtcttctgc ttgcagtgac gggcaggccc tgagtttttc 6360
tctggcacac acccatcagt taggggctgc ctctgttgtc tgctagcgcg tagtagcgcc 6420
atggccccgt gggcacccgg gagccagccc tgaggcctca ggccccagct ggcacatgct 6480
ccctggctgg cgttagcttg gttggtggcc ataggaggcc agtgggtgag gactgtcacc 6540
ttctgtgtgc ctcagagagg agactgaggc atagaggccg aggccctcgt agaggttgcg 6600
gggccaggag gctgcagagc cgggcctcgt ggaacattct ctgcctttct ggggttctgt 6660
gcttcaagga gctcagagct gtgacctgtc atgagccatt ccgtgtaggg gggcttctgg 6720
tcactggggg ccacccacat ccacagggaa caggaaagga cctctgagca aggcccctgc 6780
cccagcgggc agggctgggg aggtgggtgg agaggggttg aggcaggcct cgggagtgaa 6840
cgccagccca ggcacagggg cctctcggca gggggactgg gtcctctctg tgcctggagg 6900
gagggccctc agcagatgaa agggcaggtg gggcatgggg aactggccct ctgtcctggt 6960
tcaagcctcc tggtgtgtct catgtgtgtt gtgtgtgttg tgctgtgtct gcacgtctgc 7020
gtgtgcccgt gctctggtgt gggtgacggg gacacggtgc cgcctgcagg agctgctggc 7080
cgagtggcag ctgcggcaca gccccaggag cgtgtgcggg aggctgcggc aggcggctgt 7140
gctggggctt gtgtggctgc tgtgtctggg gaccgcgctg ggctgcgccg tggccgtcca 7200
cgtcttctcg gagttcatga tccaggtgcg gggatggggg gagcctggcc cttgcttgga 7260
acgtgtcccc agtgactctg ggcacctgcc cgctggggag gcagccttgc ctctcctcct 7320
ccacccagcc agcccagggt gccacgtacg ctgtgccccg tgctggggcc ccctccgagc 7380
cccacttctc agccccttgt cctgtctcac ggttttattt tttggtttct ggtttctttt 7440
ccactacttg taccccctccc cgcttcctgt gtggtttgaa cttgaggcct ggggagggcc 7500
ggatcttgga ggagggaggt gctacctgcc agttccaccc cgggcccttc cctgtagctg 7560
cccgctccca ccatggatgg ggggcttcca tgacaccgct ccctgcaccc ctgcagagtc 7620
cagaggctgc tggccaggag gctgtgctgc tggtcctgcc cctggtggtt ggcctcctca 7680
acctgggggc cccctacctg tgccgtgtcc tggccgccct ggagccgcat gactccccgg 7740
tactggaggt gtacgtggcc atctgcaggt gtgtggccgc acatgaaaac atcatttggt 7800
gctggagctg atactggttg tttagctccg ggtgtatcca aacctactta ggatgaggcc 7860
ggtgtgacca agcccctggt gggggccctg ggtgcatctt tctgtcccct gtacccgccc 7920
ctcagccttt gcaccccgcc ctccgcagga acctcatcct caagctggcc atcctgggga 7980
cactgtgcta ccactggctg ggccgcaggg tgggcgtcct gcagggccag tgctgggagg 8040
atttgtgggg ccaggagctg taccggttcc tggtgatgga cttcgtcctc atgttgctgg 8100
acacgctttt tggggaactg gtgtggaggt gagggggtcc tgggcggact gggtggtgag 8160
tgttctcccc ttagtggccg ggacgtgtgg ccgggaggtt ctcaggcctg gacctttccc 8220
atcagggccc tgtgtctcca cccttcagag gaagactcag gaaatagaac cgaactcatt 8280
tttagattgg aaaggaaaac aggtttcaaa gtgatgtgta tggccaggca tggtggctca 8340
tgcctgtaat cccagcactt tgggaggctg aggtgagtgg atcacctgag gtcaggagtt 8400
tgagaccagc ctggccagca tggcaaaacc ccgtctctac taaaaataca aaaattagca 8460
gggcgtggtg gcgcacacca gtagtcccag ctactcggga ggcagaggca tgagaatcgc 8520
ttgaacctgg gaagcggagg ttgtagtgag ctgagattgc gcctctgcac tccagcccgg 8580
gcgacagagc aagactgtgt tgccaaaaaa aaaaaaaaaa aaaaagtaac gtgtatatga 8640
cacaacctca gtctaggata caaaggaaaa acacacagca gggaacagac agcggaggaa 8700
catttgaaaa atgttccagg tattccgctg tgggttctgg cacttcagct gtttttttcc 8760
tcttggtgtt ttcttcgaga atatatgaat ttaataataa taaaaaataa gattaaaaaa 8820
atcagtagga ggcccggtgt gttggctcac gcctgtaatc ccagcacttt gggaggctga 8880
ggtgggagga tcacttaagc ccaggagttt gagaccagcc tcggcaacag agtgagacct 8940
tgtctctaaa gaaataaaaa taaaaataaa taaaaattag ctgggcctgg tggtgtgtgc 9000
ctgtagtccc agctactcag gaggctgagg tgggaggact gcttgatccc aggaggtcaa 9060
ggctgcagtg agccacgatt gcgtcactgt gctccagccc ggacaacaga gccagaccct 9120

gtttcaaaaa agaaaaaatc atttggtgct ggagctgata ctgcttgttt aactccgggt 9180
gtatccgaac ctacttagga tgaggctggt gtgaccaagc acctggtggg gtcccccagg 9240
ctggtggctg tctcttcttg gcattatctg tggaacggcc aggatgtccc ctggctgggg 9300
gcggggtgcc tctgggtgtg gggactcagg ctggcctccc tgggcatggc tttcccatct 9360
aacgatccgg ggtcctcgga ggcaggatta tctccgagaa gaagctgaag aggaggcgga 9420
agccggagtt tgacattgcc cggaatgtcc tggagctgat ttatgggcag actctgacct 9480
ggtgagggtc ctctgcttgc ctcctgctgt ctggcccctc ggtcagtgcc cctttccccc 9540
cgggggtcct gtacctgcct ccccctgggg ccacagccag gcccggccat ggaccctgga 9600
gtgatgcaga ccctaacccc ggggactcag ttttggccac tgtcacatgc cctgcccgtg 9660
gtctggaacc agcctgaact cggggagggg agggcactgg agcagcccct gaactgccac 9720
cccctcccca ggctgggggt gctcttctcg cccctcctcc ccgccgtgca gatcatcaag 9780
ctgctgctcg tcttctatgt caagaaggtg aggctgccaa ggcctgggga gggctgggct 9840
ggcagggtgg ccggcagtgg gggcaggact cagctgggca gagactggga cactacaacc 9900
ctccccacct gagtggaatg gggctggcgg ctcggggctg ccctggcctg tccctccgcc 9960
caagggacgg ctgacccctc ttggcatcac tgtcttcggc aggccgggaa gcatcgctgg 10020
gttcagaggt cattagcagg tacctgtgtg cacctggcca ggggtagggc cagggctctg 10080
ggagacacca agccttggga cctctgggtc cctggaaaag tgatgctggg cctctctccg 10140
caactgggtc atgcccagtg gctgagaact gggacctttc ttcccatcct ggctcctgtc 10200
tttttatttt attttatttt attttttgag acagagtctt gctttgtcgc ccaggctgga 10260
gtgcagtggc gcaatctcgg ctcactgcaa gctccgcctc ccgggttcac gccattctcc 10320
tgcctcagcc tcccgagtgg ctgggactac aggcgcccgc caccacaccc agctaatttt 10380
tttgtatttt tagtagagat ggggtttcac tgtgttagcc aggatggtct tgatctcctg 10440
accttgtgat cgcccacctc agcctcccaa agtgctggga ttacaggcat gagccaccgc 10500
acctggccat cctggctcct gtctcgtctg gccttttgtt tcctcatccc tgcagtgggg 10560
accatgttcc agtggggact gagggaggtg gtgtgtacag tgttgtgtgc agagcctggc 10620
atggcagcga ccttcagcag atggaggtgc tgttggcatc acccgtttgc gtgcaagtga 10680
ccggcaggct tctgtcttcc tctgtaaatg aggaggctgc agggagcagg gtccaggctc 10740
tgcctggacg cacagccctg gtcgcccttc cctcttgagc tcccccaggg cttggagggg 10800
tgctgagggg tagcaggatg gtggcagagc gcagcctttc cccacagacc agccttctgg 10860
ccaactgcca ggcgccgcgc cggccctggc tggcctcaca catgagcact gtcttcctca 10920
cgctgctctg cttccccgcc ttcctgggcg ccgctgtctt cctctgctac gccgtctggc 10980
agtgagtggg gcgggtggag ggagaccctt ctgtgtcatc tgggggttgg gtggtggtgc 11040
ctggggggcc caaggcttag gggtgcagag gacgggctgg gagggagccg ggtgaactgt 11100
tgctcggggt gagggttcct ggtcggcatc ttccccaggg tgaagccctc gagcacctgc 11160
ggccccttcc ggaccctgga caccatgtac gaggccggca gggtgtgggt gcgccacctg 11220
gaggcggcag gccccagggt ctcctggctg ccctgggtgc accggtacct gatggaaaac 11280
accttctttg tcttcctggt gtcagccctg ctgctgtgag tcccgcaggt gggggcggcc 11340
ctggggagat ggccctcgct gggcctgcac cgtgtgggga ctggccgctc ccgggctgtg 11400
gccctgggcc ctgcccgaca gcccctctcc cgcagggccg tgatctacct caacatccag 11460
gtggtgcggg gccagcgcaa ggtcatctgc ctgctcaagg agcagatcag caatgtgagt 11520
gcccctcctg ggctgctctc agcccccacc ctctcaggtc acctgagggt gacgctcccc 11580
tccactcccc tgtacatctc ctgtgggcct tggctggtga aagggtttgt ttgccaaaca 11640
agtgaccctg atcaatacgt gcacttcaga tcaagtgacg aagtagccgt gagacagagc 11700
caggctcctg atccccaagg acccgcaggg ccccactggg accagggctc tggggggtgg 11760
ggtgtgattt cagcccccgg gtgtggcccc gctgagggtt tagacagtct ctgtgaggat 11820
ttttgggtcc ctatggactc ttcactcatt ttacaaacag gtggattaag gttgagagtt 11880
aggacttaaa actttttttt ttggagacgg agtctcgctc tgtcacccag gctggagtgc 11940
agtggcacag tctcagctga ctgcaacctc tgcctcctgg gttcaagcaa ctgtcttgcc 12000

tcagcctccc gagtagctgg gagtacaggg cctgccgcca ctccggctac tttttgtgt 12060
ttttcgtaca gatgcagttt cactgtgttg cccaggctgg tctcgagctc ctgaggcaat 12120
ccacccacct tggcctccca aagtgctagg attataggca tgagccaccg tgcctggcca 12180
aaacattttt atgaagaaaa caatgtttga ccgggtgtgg tggggctcat gcctatagtc 12240
ccaatgcttt gggaggccaa ggtgggagaa tggctggagc ccaggagttc gagaccagcc 12300
tgggcaacag agtgaaacct cgtctctata aagattttaa aagttagctg ggtgtggtgg 12360
tgtgcacctg tagtcccagc tacttgggag gctgagggag gagattgctt gagcccaggt 12420
tgaggctgca gtgagctatg attgtgccac tgcgctccag cctgggtgac agtgaaactc 12480
tgtctcataa aaaaaaaaaa aaaaaaagcc tgggcatggg ggctcaggcc tctaatccca 12540
gcactttggg aggccgaggt gagcagatca cttgaggtca ggaggtcgag accagcctga 12600
ccatcatggc aaaaccccgt ctctactaaa agtacaaaaa tcagctgggc gtggtggcgt 12660
ccacctgtac cagctactca ggaggcggag gcaggagaat cagcccagat tgcgccactg 12720
cactccagcc cgggcgacag agcgagttgc tgaggccctc atgctgtgtc atgggagtga 12780
gttgcagaag ggggtcggac cccataccgg agaggcagcc cccaggagta ctgtccaccc 12840
ggcagctggt ccctgtgccc actcccttcg cccctgtgcc cactcccttc gcccctgtgc 12900
ccactccctt cgcccctgtg cccactccct tcgcccctgt gcccactccc ttcgcccctg 12960
tgcccactcc cttcgcccct gtgcccactc ccttcgcccc tgtgcccact cccttcgccc 13020
ctgtgcccac tcccttcgcc cctgtgccca ctcccttcgc ccctgtgccc actcccttcg 13080
cccctgtgcc cactcccttc gcccctgtgc ccactccctt cgcccctgtg cccactccct 13140
tcgcccctgt gcccactccc ttcgcccctg tgcccactcc cttcgcccct gtgcccactc 13200
ccttcgcccc tgtgcccact cccttcgccc ctgtgcccac tcccttcgcc cctgtgccca 13260
ctcccttcgc ccctgtgccc actcccttcg cccctgtgcc cactcccttc gcccctgtgc 13320
ccactccctt cgcccctgtg cccactccct tcgcccctgt gcccactccc ttcgcccctg 13380
tgcccactcc cttcgcccct gtgcccactc ccttcgcccc tgtgcccact cccttcgccc 13440
ctgtgcccac tcccttcgcc cctgtgccca ctcccttcgc ccctgtgccc actcccttcg 13500
cccctgtgcc cactcccttc gcccctgtgc ccactccctt cgcccctgtg cccactccct 13560
tcgcccctgt gcccactccc ttcgcccctg tgcccactcc cttcgcccct gtgcccactc 13620
ccttcgctcc tctctgccca ggaggacctc cttgcccact gggcacccac caagttggag 13680
acaaggcact ttccccccgg gctacccagc ctggcctcct ccccgaggcc cctcccctgg 13740
ggaacggccc cccgtgaagc aggtcccgtg gcttttccag gcttgcgtcc tgctgctggg 13800
ctctgccctc cccctagggc tccccatccc ctcccattgc catttgtcct cctgcaggtt 13860
ttcactctgg ggctggtgag tgccagtgtc cttaggcatg ctccacacca gggctagggg 13920
ggcgcttgtg gggatcagcg gctggcgtct gccaaggcga gggcagggcg gggtgcagcc 13980
agtgggcctc ggcagccatg tgaccctgct gctggctagt tggtgccagg atccagggct 14040
ccaggcagct cgtcccagct cgtcccaagc actcatggga cggctcccac attgcttggg 14100
ttgtaaacac tgtagtacac actgtcccat ctcctgcctt tcccgtttca ccacatagag 14160
ctgaaagcct ggcatctcgg ctgcggtggt tgtctggctg tagatattgg aagtggggaa 14220
tggctcctgc cctactgggt ggcatttcag agcccccagg ggccaggtag ggccaggtgg 14280
gtccgggcgg ggaatgcaga gtggtgctgg gagaatccca gctctaccca ccgttgctct 14340
gggatgggaa ggacctttct ccaggcggat gctggctgtg gtgtctaccc aaggccgagc 14400
tctgacccgt ctttggggct gtcggtctga gttttccccg tgcaggtcgg catggctcaa 14460
gtgcacagcc cctccatact catcagcaac tcgatagaag ctacttaggt tgttgcttaa 14520
ggggcccatg gtctctcctt gaaggtattc taagtggtcc tcctaggctg agccaggttg 14580
ccaggtgccc agagccaatc cgtgtggctc ttgacagaac tcctgttctc tgtgtgaagc 14640
tccgggcggg gaatcccggt ctgacccagg cactgatgat cagtgtttcg gtgcaccaga 14700
tcctttgcta agtgccccct ctgcttgtca catttcattg tcgaaagagc cctgggtcag 14760
tccagtactg gccccaaaga tgtccccgtc ctaagactgg atggagcccg tgaatacgtt 14820
acgttgcgtg gctggggaat tacgattgca ggtagaatta aagttgccag tcagctgact 14880

ttggatagga gatggcccta gattatcgtg gtggcccagc gtgatctcag ggtccttaga 14940
ggtgaaaaac agaggccgga gtgtcagggt cagagtggtg ccacgtgagc aggacttgag 15000
cagccactgc tggctttgaa gtggaggaaa gggctgagag ccaggggcct ctagaagttg 15060
gaaaaggcga ggaagaggat tctcccccga agcctccggg aggaatgtag ccctgatcat 15120
gcttgactgg agcccagtga gactcatttt ggacttctca cttccagaac tgaaagttga 15180
gaaaccggtg gtgttttaag ccacgaagtt gatgacagtt cgttacgaca gcagcaggaa 15240
actaatgtaa accctgtgcc ttgtgttatt atcccccttt acagatgctg ccactgaggt 15300
cacagagtta gtaacccaag gcagagccag tacgtggcag ggctggtttt aaattgagtc 15360
aggctggctc tgggctgctg attacatttc ccttttcact ttggccacta ggaagcttag 15420
atccacacat acgcccactt cctggctctt ggtttttcct tgtccttgtt tggtggccct 15480
ttcacctttt cttttatgtt tttgctgctt gcgatgttga atacacctcg tgagttttcc 15540
ctcaaatgca gtttggaaca ggatggctct ggatgggctg atggatgatt tctcctcccc 15600
tttgtgttct ggcctttcta ggagggtgag gacaaaatct tcttaatcaa caagcttcac 15660
tccatctacg agaggaagga gagggaggag aggagcaggt gagggtccgt ggagggattg 15720
gggacactct gagcctggag gggagagggg ccagggctgg acagtagggc tgccactggg 15780
gctacaccac gacattgctg acacttgacc gcttttgacc tacaaaaatc tcccccccgg 15840
cgcccacctt cactctctct agagccttct ctggcctccg gagcctggcc attttgacag 15900
ggacctaaaa tacatctcag gagaaaggct gtggggcgtc tggcctgtcc ctcgccgggc 15960
cttggctgtg cccagccagg ttgcagggat gttgatgttt atggggtccc ctcagtgatg 16020
tctctttctg gcagggttgg gacaaccgag gaggctgcgg caccccctgc cctgctcaca 16080
gatgaacagg atgcctag 16098

<210> 4
<211> 9524
<212> DNA
<213> human

<220>
<221> misc_feature
<222> (1)..(9524)
<223> Evin-2 gene

<400> 4
atgctgctgc cgcggtcggt gtcatcggag cgggcccctg gggtgccgga gccggaggag 60
ctgtgggagg cagagatgga gcggctgcgc ggctctggga cgcccgtgcg cgggctgccc 120
tatgccatga tggacaagcg cctcatctgg tgggtgccac gcgggcgcca gacggtgcgt 180
ggggggggtg ctgcgaggct gccccgtcgg atggtgtggg agcaccccgt ctgcttggcc 240
cgggtgggca gggcgggtga ctcaggggcg cccctgtcac cacccccgtc caggcagctg 300
cgggagcccg cgggggtgca gaccttgcgc tggcagcggt ggcagcgccg gcggcagacg 360
gtggaaaggc gcctgcggga ggcagcgcag cggctggccc ggggccttgg gctctgggag 420
ggggcgctct acgagatcgg gggtaggacc cgcgcgaccc gcacctcccc ttgccctctc 480
tggagcccca ctgcccagat cggaaaaagg agagtggcat catcccacct gccttcaccc 540
gggtcccccg accaatcggc ccctcccccc tcccacccct tccgcccgca ggcaccgcaa 600
acctcgggcc cacgcagcac ccccaggtga ctgtcagcgg tacctccgga ctcgggcggg 660
tgggagcctg gcgggcaccc cacgccgtcc ggtgggcccg cccttggact ctcagcgcgg 720
ccccagcccc ggccccggcc tccctgaccc accctgctct cccactgcag gcctcttcgg 780
cacaggaatt cggtcctact tcaccttcct ccgcttcctg ctgctactca acctgctgag 840

cctgctgctc accgcaagct tcgtgctgct gcccctggtc tggctccgcc cccctgaccc 900
aggccccacc ctgaacttga gtgagtgtga ggcccaccag gggaagtgct ccggtgccca 960
cctgcgccat gggggggctg gcccagggcc cagagcgtgg cgacaacgct gggcgtggtc 1020
ctgccgtgca ggccccgggg ctctctctcc ctgacctcgc cttgtgtggg gcacgccttt 1080
ggcacatcct cagccccctg cccaggcctc cccagggttg ggggttatag agcagtagcc 1140
gcagagcctc acctatgcct tggggatccc tctctattga cccccttcct cctcaacagc 1200
cctccagtgc cctggtagcc gccagtcccc gcctggcgtt ttgaggttcc acaatcaact 1260
ttggcatgtt ttgactggca gggtgagtga ggtctgtccc ggctatggtc cagagtctgg 1320
gagacccagg gaactggctt caggggacac aagtctaaga ggacctctgc tcctccaggg 1380
acatttcggc tcctcagggg atggtcttac ctagacagac tggcctggcc ggtggtgggg 1440
ttagagagag gctgaggcca ctgtgcccaa catagactca tggacacatg cccgtggtcc 1500
tcagagccca gctggtgtag gcggtgccag ggacaaaggg ggaaaagtgc cccggcctct 1560
gggagaagca cttccccacc ctcatcctgg gaagccatcg agtggtcacc aatggggttc 1620
ctgggttcca gtcgtggctc tatttctcaa agccctgtga ccgtgggcac gttgccgaac 1680
ctctgtgggc ccttgtaaga cgggacactc cctacctgat ggggggattg cacgggggct 1740
aacaagatca tatatggaaa gcacttgagc agagcctggt gctcacccgg gctgggtatc 1800
ttcgtcgctg tccccaggcc ttcaccaaca cctatctctt ctacggtgcg taccgagtgg 1860
ggccggagag cagctccgtg tacagcatcc gcctggccta cctcctcagc ccgctggcct 1920
gcctgctcct ctgcttctgt gggactctgc ggcggtgaga gcgaggtcca caccttcacc 1980
ccttccccag ggaatcttcc ctgatcaccc cttccttggc agggtaccct cccattggca 2040
ggaaggccca tggcccggca cagcaggctc ctgggtgctt cctcagccca ggctctggcc 2100
gcaaacctta ggaacagggc caccgccccc taccccgact cctcactcac caggacctgc 2160
acctggggct gcaggggcct tcccagaccc agagccgagc caaggctgag gctggatggt 2220
agaggtgccc ctcctccagc agcccctggt ctcctgcccc gcaggatggt gaaggggctg 2280
ccgcagaaga ctctgctggg tcagggctat caggcgcctc tcagcgccaa ggtcttctcc 2340
tcatgggact tctgcatccg ggtgcaggaa gcagccacca tcaagaagca tgagatcagc 2400
aacgagttca aggtgtgtgc acgagtgagt gcctgagatc cacaagctcc aggtatatgg 2460
gagcgagtgc gtgagagcca cgtgttccag gtgtgtgcga gcaagtgcga ccgtgccagt 2520
gtgtgtgagc gtgtgtggga gggagcctgt gggagcgtga tggagagcgt gtgaatgtgt 2580
gtgcatgtgt ggccgagtgt gagggcacat gtgactgaat gggtgatggc gagcacgcct 2640
gtgaatgtgt gaatctgagt gtctatggga gcatgacagt gtgagagttt gtgactgtgt 2700
atgtgagcgt gaatggttgt gactgtgtgt gagagcgttt cctgcaccct ttcctcccat 2760
gcccaccccg cctgcagctg cctctgtccc cacccttccg ggcaggtgga gctggaggag 2820
ggccgtcgct tccagctgat gcagcagcag acccgggccc agacggcctg ccgcctgctc 2880
tcctacctgc gggtcaacgt actcaacggg ctcctggtgg ttggggccat cagcgccatc 2940
ttctgggcta ccaagtactc acaggacaac aaggaggtgt caggcaactg cattcattta 3000
atcctggcca gaactgcggg ggtgagacag gatgatccca ttttacagat gaggaaaccg 3060
aggctcagag aggttcaatc ggtcgtggcc acaggtcacg gccaaggcag gcgggctccc 3120
actggatatt cccgccaact gccaggctgc tgcagggaat agcttacagg cgaccctatt 3180
ctgggcctgc cccagagtta cactttaggg cactgtgggg ggcggggagc agacaggggc 3240
cccccagcac gcgggctccc ctgacctgcc ttttcccgca ggagtccctg tttctgctgc 3300
tccagtacct gccccctggg gtcatcgccc tggtcaactt cctgggtccc ctgctgttca 3360
catttctggt ccagctggag aactaccctc ccaacacgga ggtcaacctc actctgatct 3420
ggtgagtgcc acccttggtg gggacaagtg ggcatgtaac aagtctgcat cctgctgctc 3480
tcttttggtt gtcacctggc acctggggtc cccagctgag aggcttcccc cttcactagt 3540
ggcccaaggg aaggcaggta cacacctcac agcacacgac ctcagggccc caggtctaag 3600
gctttccaat cccccacctc caaaggaagc cgccagtctg gaccaagctc cggccattca 3660
gtggagcaag ggactgtggc caggggtgct gggaatgggc tttgcacaca ggaaaaccag 3720

caacacggga catgcagagc ttctacatgc cgagggttta ctccccttac ctggacaacc 3780
ctcacgtgac ccgtgaagtc cagaccatcc tcatccccct tgagggatga ggaaactgag 3840
gttcacatga gtgaagcgcc cagccaaagt caagctcatc gtgcctgcca ggtgcaagtt 3900
caaaagcaag ccctcgacct cctgggctca agcgatcctc ccgccggagc agctgggact 3960
accactccca ggttctgcca tcttctgtct cagcacttag gtcctgtcta gaccccctac 4020
ttctccaccc ctcatccagc tgcagaaatt cttgtgtaaa gcccacctga gctcccactg 4080
gcttctctcc ctcacgccac cctcagtcac cacttgccct aactgctttt ccctctgctt 4140
cctcaaagtc agctgtgttt ggtttgcttt gaaactttat tttttgaggc cgggcatggt 4200
ggctcacgcc tgtaatccca gaactttggg aggccaaggt ggatcacctg aggtcaggag 4260
ttcgagacca gcctggccaa catggtgaaa ccctgtctct actaaaaaca caaaaatcag 4320
ccaggtgtgg tggtgcacac ctgtaatccc agctacccgg gaagctgagg caggagaatc 4380
actcgaaccc aggagacaga tgttgcagtg agcagagatt gcgccactac actccagcct 4440
gggtgacaga gtgtgacttc atctcaaaaa agaaaacaaa acaaaaaaag ctctcaccta 4500
cccctagctc cctcaacagg agaaccactg ttcccagcca ctctgtagcc ccatatcagc 4560
agtgggaatg ttcaccctta gagataacac gcaaccaggc acagtgactc acgcctgtaa 4620
aggccaacac tttggaaggc caagatggga gaatcacttg atcccaggag tttgagaccg 4680
gcctgggcaa catggcaaga ccccgtctct acaaaaaata caaaaaaatt aactgggcat 4740
gatgaaagac taggacacac agtcctagct actagagaag ctgaggtggg aggatcactt 4800
aagcccagga ggtcaaggct acagggagct gtgatcgcgt cactgcactc cagcctaggc 4860
aatggagtga gaccctatct caaaaaaaaa aagcaaaaac agataacatg catgtgatga 4920
tgtttcgttt agacaatcac ctatttggtc tggtcatgca ttgtgcagta catatgtgtg 4980
gcacgtgttt atgacatgca agcttgtgtc agtgggcttc aatgtccata ggcggcggag 5040
gaaaggagga gctcgcctgc agtcaggatc ctgaaaaggc cacaggcttc tccaccgcag 5100
ggcttgctgc atgtgcctct ccacaccagg catgagggaa catggctgga ctgtgcagtg 5160
tttctcaaac tgatctaaaa acagaaccct tggccaggcg ccgtggccca cacctgtaat 5220
cccagcactt tgggaggcca aggcgggtgg atcacctgag gtcaggagtt cgagaccagc 5280
ctggccaaca tggcgaaacc ctgtctgtac tcaaaataca aaatttagtc agaaatcgct 5340
tgaacccagg aggcagaggt ggcagtgagc cgaggtcaca acactgaact cctgcctggg 5400
tgacagagtg agactcggtc ttgaaacaaa acgaaaacac agaacccttt ttcccatgaa 5460
tcgcctggag actcggactc tgagaacgca ctttggacca cattgccaca gacagagcag 5520
gttggaagga ggctgggccc aggacacaac atgatcccac tcggacatca gccacaccta 5580
tagcctgggt agagcccggt gcccatgtgc ctgggcccct ccacctgaca aggtccctgc 5640
ccccaggtgc gtggtgctga agctggccag cttggggatg ttctccgtct ccctgggtca 5700
gaccatactg tgcattggca gagacaagag cagctgtgag tcctacggct acaacgtttg 5760
tgactatcag gtggctggca gcccggcggg gcctgtccct cccttccttc tccccaaaaa 5820
gctcacctca aactgtgcag agccctgttc ctgccccttt agtcaccttt gagagaagct 5880
gtgagcaggg ctgcctgcac cgccgcacac ctccaggggg cgccactgct gccacatggt 5940
gtagtgtggc tgccctggct gtggggcttc ctgctctaat cccatcctca aggcctgggt 6000
ttcaacctaa cgattctatc agacaccagg caaggcacct gcaccacaga aaccctcaca 6060
ggaggctaag ggaaggaagg gccggggtgg ccgtgagtgg tgacgggtcc ccttcccctg 6120
caccccagtg ctgggagaac tccgtggggg aggagctgta caagctgagt atcttcaact 6180
tcctcctcac cgtggccttc gccttcctgg tcaccctgcc tcggaggtga gccccggggt 6240
gacacctcca gaagggcggg ggtgccgcga gcatgttggg ggtggccagt ggctacagcc 6300
aagggtgagc gggtccagtg tggagcccgg gagtaagtgg caggctgtgg ccatgaccaa 6360
tacagcccac gcatctgtct ggagtggtga gtacctggac cctcccatcc ctgcaggctg 6420
ctggtggacc ggttctcagg ccggttctgg gcctggctgg aacggaggga gttcctggtc 6480
cccaagaatg tgctggacat cgtggcgggg cagacggtca cctggatggg cctcttctac 6540
tgccccctgc tgcccctgct gaatagcgtc ttcctcttcc tcaccttcta catcaagaag 6600

gtgacggctc atggctgggg ggtatggggt tcgtgcctct gggtggatgc cttgagctgg 6660
gctcgcctcc tgctcctgct cctgccccct tccctgggtg tcagccccct ggggtctgca 6720
taactcgctg actcctggtt gctattgctt gcgcccccag tacaccctcc tgaagaactc 6780
cagggcatct tcgcggccct tccgtgcctc cagctccacc ttcttcttcc agctagtgct 6840
cctcctgggc ctgcttctgg ctgcagtgcc cctgggctat gtggtcagca ggtgagggga 6900
agaggagggg ggcacccaga ggctggcagg ggcagtttct cacccaggac cctgatgcca 6960
gccccacttg gccatctctc gccagcatcc actcctcctg ggactgcggc ctcttcacca 7020
actactcagc accctggcaa gtggtcccgg agctggtggc ccttgggctc ccgcccattg 7080
gccagcgtgc cctccactac ctgggctccc acgccttcag cttccccctc ctcatcatgc 7140
tcaggttctc agggcagccg gggccatggg aggggacacc tggaggggga ggtccttcct 7200
tccatggggg tggtgagcct gtgcaccccc aaccaggagc cagacgcaga aagccaaggg 7260
aagcaggggc ctctgaaaag caggaacctc ctgggcccac cctctgggct gccatgggga 7320
ttgcaggatc actggggaag caccgtgtct gggtggctgc agctgctgag ctattggtat 7380
tgctgtgcca gaggggaggg gatgaggggc tctgcgagga aggagaggag ggtcccatcc 7440
tcagatacag cagtgtgcag tgagaagacc ccagtaccgc gtattgtaga gattgaggtg 7500
gggagagagg aagtcaggga gaggcgcctg tggccccagg ggcaactgac cacgaatccc 7560
cttcccaccc aagccttgtc ctgacggtgt gcgtctccca gacccaggcc aatgccaggg 7620
ccatccacag gctccggaag cagctggtgt gggtgagtgt cctcggggct ggtgagggga 7680
cagcagcttc agtggaaacc cttccctatg tgtggccgag ggcctagaac acgtctgagc 7740
gggtcaggtg ggttcttccc actggagggc gtggcctcag gctgagagtg aagacgggga 7800
aggggaggaa gagaacagct cgggctcctg agaccaggag ccagacctgg taagtacatg 7860
accttagggg ctgggccttt gcctgtaatc ccaacgcttt ggaggcccag gcaggaggat 7920
cggatcactt gaagccatga gttaaaacca gcctgggcaa caaagcaaga ccctggtctc 7980
caccaaaaat aagtaattaa ttttttaaaa ggagaatgtg gccgggcgtg gtgactcacg 8040
cctgtaatcc cagcactttc agaggccgag gtgggtggat cacctgaggt caggagttca 8100
agaccagcct ggccaaaata gcgaaacccc gtctctactt aaaaaaaaaa aaaaaaaaaa 8160
aaaaaccggg cacggtggtt cgtgcctgta atccagcact ttgggaggcc gaggtgagtg 8220
gattgcctga ggtcaggggt tcaagaccag cctggccaac atagtgaaac cccgtctgta 8280
ctaaaaatac aaaaaattag ctgggcgtga tggtgggcgc ctgtaatccc agctactagg 8340
gaggctgagg caggagaatc gtttaaactc aggaggcgga ggggcggagg ttgcagtgag 8400
ctgagaccgc cccattgcac tccagcctgg gcaacaagag caaaactccg tctcaaaaaa 8460
ggaaaaaagg ataacatggc tgagtgcagt ggctcacgcc gaggcgtgaa gagtgcttga 8520
gttcaggagt ttgagaccag gctgagcaac atagcaagac ctcgtctcta ctaaaaatca 8580
aataaatgag ctgggcgtgg tggcgcgtgc ctgtggtcac agctattccg gaggctgagg 8640
tgggaagatc gcttgagcct gagaggtcca ggctgcagtg ggctgtggtc ttgctactgc 8700
aatcctgcct gggcaacagg gagacaccca gtttccacaa aaaaaaaaaa aaattacgac 8760
tttaggcttg gggccagggg cttaatggag ctgagggagg aacagtgacc tggctaaggc 8820
gtggcctcag ggtggggcct ctaccggggg cgtggccttg ggctgcttgg ggacggactc 8880
ttagcgggcg tggtctagat ctgcatgggg tgggacccta gtggggcgtg gtctcagggt 8940
gcgttggggg cggggccctt ggaggtcgtg gtctcgggcg gggacgtgga aggagtggca 9000
tctggctgcg ttgtggacgg gactcctgag ggcgtggtct agggctgcat cggggcggga 9060
ccctggtggg gcgtggccct gggagggtgt ggcctcgagc ggggcgtggc ctcgggcggg 9120
gcgtggcctc tggctacttt gggtgcgggc tggcccatgg gccgcagagt tgctgccgct 9180
gctgtcctca cacccgctcg tgggaagcag cgcctgtcgc aactcgccac ttgttctcct 9240
cacagcaggt tcaggagaag tggcacctgg tggaggacct gtcgcgactg ctgccggagc 9300
caggcccgag cgactctccg ggccccaagt accctgcctc ccaagcttcg cgcccgcagt 9360
ccttctgccc cggatgccca tgccctggct ccccgggcca ccaggccccg cggccgggcc 9420
cctccgtcgt ggatgccgcg ggactgcgtt ccccttgccc tggacagcac ggtgccccgg 9480

cctccgcccg cagattccgc ttccccagcg gcgcggagct gtaa                    9524

<210> 5
<211> 806
<212> PRT
<213> human

<400> 5
Met Ala Gln Pro Leu Ala Phe Ile Leu Asp Val Pro Glu Thr Pro Gly
1               5                   10                  15

Asp Gln Gly Gln Gly Pro Ser Pro Tyr Asp Glu Ser Glu Val His Asp
            20                  25                  30

Ser Phe Gln Gln Leu Ile Gln Glu Gln Ser Gln Cys Thr Ala Gln Glu
        35                  40                  45

Gly Leu Glu Leu Gln Gln Arg Glu Arg Glu Val Thr Gly Ser Ser Gln
    50                  55                  60

Gln Thr Leu Trp Arg Pro Glu Gly Thr Gln Ser Thr Ala Thr Leu Arg
65                  70                  75                  80

Ile Leu Ala Ser Met Pro Ser Arg Thr Ile Gly Arg Ser Arg Gly Ala
                85                  90                  95

Ile Ile Ser Gln Tyr Tyr Asn Arg Thr Val Gln Leu Arg Cys Arg Ser
            100                 105                 110

Ser Arg Pro Leu Leu Gly Asn Phe Val Arg Ser Ala Arg Pro Ser Leu
        115                 120                 125

Arg Leu Tyr Asp Leu Glu Leu Asp Pro Thr Ala Leu Glu Glu Glu Glu
    130                 135                 140

Lys Gln Ser Leu Leu Val Lys Glu Leu Gln Ser Leu Ala Val Ala Gln
145                 150                 155                 160

Arg Asp His Met Leu Arg Gly Met Pro Leu Ser Leu Ala Glu Lys Arg
                165                 170                 175

Ser Leu Arg Glu Lys Ser Arg Thr Pro Arg Gly Lys Trp Arg Gly Gln
            180                 185                 190

Pro Gly Ser Gly Gly Val Cys Ser Cys Cys Gly Arg Leu Arg Tyr Ala
        195                 200                 205

Cys Val Leu Ala Leu His Ser Leu Gly Leu Ala Leu Leu Ser Ala Leu
    210                 215                 220

Gln Ala Leu Met Pro Trp Arg Tyr Ala Leu Lys Arg Ile Gly Gly Gln
225                 230                 235                 240

Phe Gly Ser Ser Val Leu Ser Tyr Phe Leu Phe Leu Lys Thr Leu Leu
                245                 250                 255

Ala Phe Asn Ala Leu Leu Leu Leu Leu Leu Val Ala Phe Ile Met Gly
            260                 265                 270

Pro Gln Val Ala Phe Pro Pro Ala Leu Pro Gly Pro Ala Pro Val Cys
        275                 280                 285

Thr Gly Leu Glu Leu Leu Thr Gly Ala Gly Cys Phe Thr His Thr Val
    290                 295                 300

Met Tyr Tyr Gly His Tyr Ser Asn Ala Thr Leu Asn Gln Pro Cys Gly
305                 310                 315                 320

Ser Pro Leu Asp Gly Ser Gln Cys Thr Pro Arg Val Gly Gly Leu Pro
                325                 330                 335

Tyr Asn Met Pro Leu Ala Tyr Leu Ser Thr Val Gly Val Ser Phe Phe
            340                 345                 350

Ile Thr Cys Ile Thr Leu Val Tyr Ser Met Ala His Ser Phe Gly Glu
        355                 360                 365

Ser Tyr Arg Val Gly Ser Thr Ser Gly Ile His Ala Ile Thr Val Phe
    370                 375                 380

Cys Ser Trp Asp Tyr Lys Val Thr Gln Lys Arg Ala Ser Arg Leu Gln
385                 390                 395                 400

Gln Asp Asn Ile Arg Thr Arg Leu Lys Glu Leu Leu Ala Glu Trp Gln
                405                 410                 415

Leu Arg His Ser Pro Arg Ser Val Cys Gly Arg Leu Arg Gln Ala Ala
            420                 425                 430

Val Leu Gly Leu Val Trp Leu Leu Cys Leu Gly Thr Ala Leu Gly Cys
        435                 440                 445

Ala Val Ala Val His Val Phe Ser Glu Phe Met Ile Gln Ser Pro Glu
    450                 455                 460

Ala Ala Gly Gln Glu Ala Val Leu Leu Val Leu Pro Leu Val Val Gly
465                 470                 475                 480

Leu Leu Asn Leu Gly Ala Pro Tyr Leu Cys Arg Val Leu Ala Ala Leu
                485                 490                 495

Glu Pro His Asp Ser Pro Val Leu Glu Val Tyr Val Ala Ile Cys Arg
            500                 505                 510

Asn Leu Ile Leu Lys Leu Ala Ile Leu Gly Thr Leu Cys Tyr His Trp
        515                 520                 525

Leu Gly Arg Arg Val Gly Val Leu Gln Gly Gln Cys Trp Glu Asp Phe
    530                 535                 540

Val Gly Gln Glu Leu Tyr Arg Phe Leu Val Met Asp Phe Val Leu Met
545                 550                 555                 560

Leu Leu Asp Thr Leu Phe Gly Glu Leu Val Trp Arg Ile Ile Ser Glu
                565                 570                 575

Lys Lys Leu Lys Arg Arg Arg Lys Pro Glu Phe Asp Ile Ala Arg Asn
            580                 585                 590

Val Leu Glu Leu Ile Tyr Gly Gln Thr Leu Thr Trp Leu Gly Val Leu
        595                 600                 605

Phe Ser Pro Leu Leu Pro Ala Val Gln Ile Ile Lys Leu Leu Leu Val
    610                 615                 620

Phe Tyr Val Lys Lys Thr Ser Leu Leu Ala Asn Cys Gln Ala Pro Arg
625                 630                 635                 640

Arg Pro Trp Leu Ala Ser His Met Ser Thr Val Phe Leu Thr Leu Leu
                645                 650                 655

Cys Phe Pro Ala Phe Leu Gly Ala Ala Val Phe Leu Cys Tyr Ala Val
            660                 665                 670

Trp Gln Val Lys Pro Ser Ser Thr Cys Gly Pro Phe Arg Thr Leu Asp
        675                 680                 685

Thr Met Tyr Glu Ala Gly Arg Val Trp Val Arg His Leu Glu Ala Ala
    690                 695                 700

Gly Pro Arg Val Ser Trp Leu Pro Trp Val His Arg Tyr Leu Met Glu
705                 710                 715                 720

Asn Thr Phe Phe Val Phe Leu Val Ser Ala Leu Leu Leu Ala Val Ile
                725                 730                 735

Tyr Leu Asn Ile Gln Val Val Arg Gly Gln Arg Lys Val Ile Cys Leu
            740                 745                 750

Leu Lys Glu Gln Ile Ser Asn Glu Gly Glu Asp Lys Ile Phe Leu Ile
        755                 760                 765

Asn Lys Leu His Ser Ile Tyr Glu Arg Lys Glu Arg Glu Glu Arg Ser
    770                 775                 780

Arg Val Gly Thr Thr Glu Glu Ala Ala Ala Pro Pro Ala Leu Leu Thr
785                 790                 795                 800

Asp Glu Gln Asp Ala Glx
                805

<210> 6
<211> 766
<212> PRT
<213> human

<400> 6
Pro Arg Thr Ile Asn Glu Glu Val Ile Asn Met Leu Leu Pro Arg Ser
  1               5                  10                  15

Val Ser Ser Glu Arg Ala Pro Gly Val Pro Glu Pro Glu Glu Leu Trp
             20                  25                  30

Glu Ala Glu Met Glu Arg Leu Arg Gly Ser Gly Thr Pro Val Arg Gly
         35                  40                  45

Leu Pro Tyr Ala Met Met Asp Lys Arg Leu Ile Trp Gln Leu Arg Glu
     50                  55                  60

Pro Ala Gly Val Gln Thr Leu Arg Trp Gln Arg Trp Gln Arg Arg Arg
 65                  70                  75                  80

Gln Thr Val Glu Arg Arg Leu Arg Glu Ala Ala Gln Arg Leu Ala Arg
                 85                  90                  95

Gly Leu Gly Leu Trp Glu Gly Ala Leu Tyr Glu Ile Gly Gly Leu Phe
            100                 105                 110

Gly Thr Gly Ile Arg Ser Tyr Phe Thr Phe Leu Arg Phe Leu Leu Leu

115 : 120 125 :

Leu Asn Leu Leu Ser Leu Leu Leu Thr Ala Ser Phe Val Leu Leu Pro
130 135 140

Leu Val Trp Leu Arg Pro Pro Asp Pro Gly Pro Thr Leu Asn Leu Thr
145 150 155 160

Leu Gln Cys Pro Gly Ser Arg Gln Ser Pro Pro Gly Val Leu Arg Phe
165 170 175

His Asn Gln Leu Trp His Val Leu Thr Gly Arg Ala Phe Thr Asn Thr
180 185 190

Tyr Leu Phe Tyr Gly Ala Tyr Arg Val Gly Pro Glu Ser Ser Ser Val
195 200 205

Tyr Ser Ile Arg Leu Ala Tyr Leu Leu Ser Pro Leu Ala Cys Leu Leu
210 215 220

Leu Cys Phe Cys Gly Thr Leu Arg Arg Met Val Lys Gly Leu Pro Gln
225 230 235 240

Lys Thr Leu Leu Gly Gln Gly Tyr Gln Ala Pro Leu Ser Ala Lys Val
245 250 255

Phe Ser Ser Trp Asp Phe Cys Ile Arg Val Gln Glu Ala Ala Thr Ile
260 265 270

Lys Lys His Glu Ile Ser Asn Glu Phe Lys Val Glu Leu Glu Glu Gly
275 280 285

Arg Arg Phe Gln Leu Met Gln Gln Gln Thr Arg Ala Gln Thr Ala Cys
290 295 300

Arg Leu Leu Ser Tyr Leu Arg Val Asn Val Leu Asn Gly Leu Leu Val
305 310 315 320

Val Gly Ala Ile Ser Ala Ile Phe Trp Ala Thr Lys Tyr Ser Gln Asp
325 330 335

Asn Lys Glu Val Ser Gly Asn Cys Ile His Leu Ile Leu Ala Arg Thr
340 345 350

Ala Gly Glu Ser Leu Phe Leu Leu Leu Gln Tyr Leu Pro Pro Gly Val
355 360 365

Ile Ala Leu Val Asn Phe Leu Gly Pro Leu Leu Phe Thr Phe Leu Val

370 375 380

Gln Leu Glu Asn Tyr Pro Pro Asn Thr Glu Val Asn Leu Thr Leu Ile
385 390 395 400

Trp Cys Val Val Leu Lys Leu Ala Ser Leu Gly Met Phe Ser Val Ser
405 410 415

Leu Gly Gln Thr Ile Leu Cys Ile Gly Arg Asp Lys Ser Ser Cys Glu
420 425 430

Ser Tyr Gly Tyr Asn Val Cys Asp Tyr Gln Cys Trp Glu Asn Ser Val
435 440 445

Gly Glu Glu Leu Tyr Lys Leu Ser Ile Phe Asn Phe Leu Leu Thr Val
450 455 460

Ala Phe Ala Phe Leu Val Thr Leu Pro Arg Arg Leu Leu Val Asp Arg
465 470 475 480

Phe Ser Gly Arg Phe Trp Ala Trp Leu Glu Arg Glu Glu Phe Leu Val
485 490 495

Pro Lys Asn Val Leu Asp Ile Val Ala Gly Gln Thr Val Thr Trp Met
500 505 510

Gly Leu Phe Tyr Cys Pro Leu Leu Pro Leu Leu Asn Ser Val Phe Leu
515 520 525

Phe Leu Thr Phe Tyr Ile Lys Lys Val Thr Ala His Gly Trp Gly Tyr
530 535 540

Thr Leu Leu Lys Asn Ser Arg Ala Ser Ser Arg Pro Phe Arg Ala Ser
545 550 555 560

Ser Ser Thr Phe Phe Phe Gln Leu Val Leu Leu Leu Gly Leu Leu Leu
565 570 575

Ala Ala Val Pro Leu Gly Tyr Val Val Ser Ser Pro Thr Trp Pro Ser
580 585 590

Leu Ala Ser Ile His Ser Ser Trp Asp Cys Gly Leu Phe Thr Asn Tyr
595 600 605

Ser Ala Pro Trp Gln Val Val Pro Glu Leu Val Ala Leu Gly Leu Pro
610 615 620

Pro Ile Gly Gln Arg Ala Leu His Tyr Leu Gly Ser His Ala Phe Ser

625                 630                 635                 640

Phe Pro Leu Leu Ile Met Leu Ser Leu Val Leu Thr Val Cys Val Ser
                645                 650                 655

Gln Thr Gln Ala Asn Ala Arg Ala Ile His Arg Leu Arg Lys Gln Leu
            660                 665                 670

Val Trp Gln Val Gln Glu Lys Trp His Leu Val Glu Asp Leu Ser Arg
        675                 680                 685

Leu Leu Pro Glu Pro Gly Pro Ser Asp Ser Pro Gly Pro Lys Tyr Pro
    690                 695                 700

Ala Ser Gln Ala Ser Arg Pro Gln Ser Phe Cys Pro Gly Cys Pro Cys
705                 710                 715                 720

Pro Gly Ser Pro Gly His Gln Ala Pro Arg Pro Gly Pro Ser Val Val
                725                 730                 735

Asp Ala Ala Gly Leu Arg Ser Pro Cys Pro Gly Gln His Gly Ala Pro
            740                 745                 750

Ala Ser Ala Arg Arg Phe Arg Phe Pro Ser Gly Ala Glu Leu
        755                 760                 765

<210> 7
<211> 2420
<212> DNA
<213> human

<220>
<221> misc_feature
<222> (1)..(2420)
<223> Evin-1  mutation C280T

<400> 7
atggcccagc cactggcctt catcctcgat gtccctgaga ccccagggga ccagggccag 60
ggccccagcc cctatgatga aagcgaagtg cacgactcct tccagcagct catccaggag 120
cagagccagt gcacggccca ggaggggctg gagctgcagc agagagagcg ggaggtgaca 180
ggaagtagcc agcagacact ctggcggccc gagggcaccc agagcacggc cacactccgc 240
atcctggcca gcatgcccag ccgcaccatt ggccgcagct gaggtgccat catctcccag 300
tactacaacc gcacggtgca gcttcggtgc aggagcagcc ggcccctgct cgggaacttt 360
gtccgctccg cccggcccag cctccgcctg tacgacctgg agctggaccc cacggccctg 420
gaggaggagg agaagcagag cctcctggtg aaggagctcc agagcctggc agtggcacag 480
cgggaccaca tgcttcgcgg gatgccctta agcctggctg agaaacgcag cctgcgagag 540

aagagcagga ccccgagggg gaagtggagg ggccagccgg gcagcggcgg ggtctgctcc 600
tgctgtggcc ggctcagata tgcctgcgtg ctggccttgc acagcctggg cctggcgctg 660
ctctccgccc tgcaggccct gatgccgtgg cgctacgccc tgaagcgcat cgggggccag 720
ttcggctcca gcgtgctctc ctacttcctc tttctcaaga ccctgctggc tttcaatgcc 780
ctcctgctgc tgctgctggt ggccttcatc atgggccctc aggtcgcctt cccacccgcc 840
ctgccgggcc ctgcccccgt ctgcacaggc ctggagctcc tcacaggcgc gggttgcttc 900
acccacaccg tcatgtacta cggccactac agtaacgcca cgctgaacca gccgtgtggc 960
agccccctgg atggcagcca gtgcacaccc agggtgggtg gcctgcccta caacatgccc 1020
ctggcctacc tctccactgt gggcgtgagc ttctttatca cctgcatcac cctggtgtac 1080
agcatggctc actctttcgg ggagagctac cgggtgggca gcacctctgg catccacgcc 1140
atcaccgtct tctgctcctg ggactacaag gtgacgcaga agcgggcctc ccgcctccag 1200
caggacaata ttcgcacccg gctgaaggag ctgctggccg agtggcagct gcggcacagc 1260
cccaggagcg tgtgcgggag gctgcggcag gcggctgtgc tggggcttgt gtggctgctg 1320
tgtctgggga ccgcgctggg ctgcgccgtg gccgtccacg tcttctcgga gttcatgatc 1380
cagagtccag aggctgctgg ccaggaggct gtgctgctgg tcctgcccct ggtggttggc 1440
ctcctcaacc tgggggcccc ctacctgtgc cgtgtcctgg ccgccctgga gccgcatgac 1500
tccccggtac tggaggtgta cgtggccatc tgcaggaacc tcatcctcaa gctggccatc 1560
ctggggacac tgtgctacca ctggctgggc cgcagggtgg gcgtcctgca gggccagtgc 1620
tgggaggatt ttgtgggcca ggagctgtac cggttcctgg tgatggactt cgtcctcatg 1680
ttgctggaca cgctttttgg ggaactggtg tggaggatta tctccgagaa gaagctgaag 1740
aggaggcgga agccggagtt tgacattgcc cggaatgtcc tggagctgat ttatgggcag 1800
actctgacct ggctgggggt gctcttctcg cccctcctcc ccgccgtgca gatcatcaag 1860
ctgctgctcg tcttctatgt caagaagacc agccttctgg ccaactgcca ggcgccgcgc 1920
cggccctggc tggcctcaca catgagcact gtcttcctca cgctgctctg cttccccgcc 1980
ttcctgggcg ccgctgtctt cctctgctac gccgtctggc aggtgaagcc ctcgagcacc 2040
tgcggcccct tccggaccct ggacaccatg tacgaggccg gcagggtgtg ggtgcgccac 2100
ctggaggcgg caggccccag ggtctcctgg ctgccctggg tgcaccggta cctgatggaa 2160
aacaccttct ttgtcttcct ggtgtcagcc ctgctgctgg ccgtgatcta cctcaacatc 2220
caggtggtgc ggggccagcg caaggtcatc tgcctgctca aggagcagat cagcaatgag 2280
ggtgaggaca aaatcttctt aatcaacaag cttcactcca tctacgagag gaaggagagg 2340
gaggagagga gcagggttgg gacaaccgag gaggctgcgg caccccctgc cctgctcaca 2400
gatgaacagg atgcctaggg 2420

<210> 8
<211> 2420
<212> DNA
<213> human

<220>
<221> misc_feature
<222> (1)..(2420)
<223> Evin-1 mutation G1726T

<400> 8
atggcccagc cactggcctt catcctcgat gtccctgaga ccccagggga ccagggccag 60
ggccccagcc cctatgatga aagcgaagtg cacgactcct tccagcagct catccaggag 120
cagagccagt gcacggccca ggaggggctg gagctgcagc agagagagcg ggaggtgaca 180

ggaagtagcc agcagacact ctggcggccc gagggcaccc agagcacggc cacactccgc 240
atcctggcca gcatgcccag ccgcaccatt ggccgcagcc gaggtgccat catctcccag 300
tactacaacc gcacggtgca gcttcggtgc aggagcagcc ggcccctgct cgggaacttt 360
gtccgctccg cccggcccag cctccgcctg tacgacctgg agctggaccc cacggccctg 420
gaggaggagg agaagcagag cctcctggtg aaggagctcc agagcctggc agtggcacag 480
cgggaccaca tgcttcgcgg gatgccctta agcctggctg agaaacgcag cctgcgagag 540
aagagcagga ccccgagggg gaagtggagg ggccagccgg gcagcggcgg ggtctgctcc 600
tgctgtggcc ggctcagata tgcctgcgtg ctggccttgc acagcctggg cctggcgctg 660
ctctccgccc tgcaggccct gatgccgtgg cgctacgccc tgaagcgcat cgggggccag 720
ttcggctcca gcgtgctctc ctacttcctc tttctcaaga ccctgctggc tttcaatgcc 780
ctcctgctgc tgctgctggt ggccttcatc atgggccctc aggtcgcctt cccacccgcc 840
ctgccgggcc ctgcccccgt ctgcacaggc ctggagctcc tcacaggcgc gggttgcttc 900
acccacaccg tcatgtacta cggccactac agtaacgcca cgctgaacca gccgtgtggc 960
agccccctgg atggcagcca gtgcacaccc agggtgggtg gcctgcccta caacatgccc 1020
ctggcctacc tctccactgt gggcgtgagc ttctttatca cctgcatcac cctggtgtac 1080
agcatggctc actctttcgg ggagagctac cgggtgggca gcacctctgg catccacgcc 1140
atcaccgtct tctgctcctg ggactacaag gtgacgcaga agcgggcctc ccgcctccag 1200
caggacaata ttcgcacccg gctgaaggag ctgctggccg agtggcagct gcggcacagc 1260
cccaggagcg tgtgcgggag gctgcggcag gcggctgtgc tggggcttgt gtggctgctg 1320
tgtctgggga ccgcgctggg ctgcgccgtg gccgtccacg tcttctcgga gttcatgatc 1380
cagagtccag aggctgctgg ccaggaggct gtgctgctgg tcctgcccct ggtggttggc 1440
ctcctcaacc tgggggcccc ctacctgtgc cgtgtcctgg ccgccctgga gccgcatgac 1500
tccccggtac tggaggtgta cgtggccatc tgcaggaacc tcatcctcaa gctggccatc 1560
ctggggacac tgtgctacca ctggctgggc cgcagggtgg gcgtcctgca gggccagtgc 1620
tgggaggatt ttgtgggcca ggagctgtac cggttcctgg tgatggactt cgtcctcatg 1680
ttgctggaca cgctttttgg ggaactggtg tggaggatta tctcctagaa gaagctgaag 1740
aggaggcgga agccggagtt tgacattgcc cggaatgtcc tggagctgat ttatgggcag 1800
actctgacct ggctgggggt gctcttctcg cccctcctcc ccgccgtgca gatcatcaag 1860
ctgctgctcg tcttctatgt caagaagacc agccttctgg ccaactgcca ggcgccgcgc 1920
cggccctggc tggcctcaca catgagcact gtcttcctca cgctgctctg cttccccgcc 1980
ttcctgggcg ccgctgtctt cctctgctac gccgtctggc aggtgaagcc ctcgagcacc 2040
tgcggcccct tccggaccct ggacaccatg tacgaggccg gcagggtgtg ggtgcgccac 2100
ctggaggcgg caggccccag ggtctcctgg ctgccctggg tgcaccggta cctgatggaa 2160
aacaccttct ttgtcttcct ggtgtcagcc ctgctgctgg ccgtgatcta cctcaacatc 2220
caggtggtgc ggggccagcg caaggtcatc tgcctgctca aggagcagat cagcaatgag 2280
ggtgaggaca aaatcttctt aatcaacaag cttcactcca tctacgagag gaaggagagg 2340
gaggagagga gcagggttgg gacaaccgag gaggctgcgg caccccctgc cctgctcaca 2400
gatgaacagg atgcctaggg 2420

<210> 9
<211> 2389
<212> DNA
<213> human

<220>
<221> misc_feature
<222> (1)..(2389)

<223> Evin-2 mutation G1129T

<400> 9
atgctgctgc cgcggtcggt gtcatcggag cgggcccctg gggtgccgga gccggaggag 60
ctgtgggagg cagagatgga gcggctgcgc ggctctggga cgcccgtgcg cgggctgccc 120
tatgccatga tggacaagcg cctcatctgg cagctgcggg agcccgcggg ggtgcagacc 180
ttgcgctggc agcggtggca gcgccggcgg cagacggtgg aaaggcgcct gcgggaggca 240
gcgcagcggc tggcccgggg ccttgggctc tgggaggggg cgctctacga gatcgggggc 300
ctcttcggca caggaattcg gtcctacttc accttcctcc gcttcctgct gctactcaac 360
ctgctgagcc tgctgctcac cgcaagcttc gtgctgctgc ccctggtctg gctccgcccc 420
cctgacccag gccccaccct gaacttgacc ctccagtgcc ctggtagccg ccagtccccg 480
cctggcgttt tgaggttcca caatcaactt tggcatgttt tgactggcag ggccttcacc 540
aacacctatc tcttctacgg tgcgtaccga gtggggccgg agagcagctc cgtgtacagc 600
atccgcctgg cctacctcct cagcccgctg gcctgcctgc tcctctgctt ctgtgggact 660
ctgcggcgga tggtgaaggg gctgccgcag aagactctgc tgggtcaggg ctatcaggcg 720
cctctcagcg ccaaggtctt ctcctcatgg gacttctgca tccgggtgca ggaagcagcc 780
accatcaaga agcatgagat cagcaacgag ttcaaggtgg agctggagga gggccgtcgc 840
ttccagctga tgcagcagca gacccgggcc cagacggcct gccgcctgct ctcctacctg 900
cgggtcaacg tactcaacgg gctcctggtg gttggggcca tcagcgccat cttctgggct 960
accaagtact cacaggacaa caaggaggtg tcaggcaact gcattcattt aatcctggcc 1020
agaactgcgg gggagtccct gtttctgctg ctccagtacc tgccccctgg ggtcatcgcc 1080
ctggtcaact tcctgggtcc cctgctgttc acatttctgg tccagctgta gaactaccct 1140
cccaacacgg aggtcaacct cactctgatc tggtgcgtgg tgctgaagct ggccagcttg 1200
gggatgttct ccgtctccct gggtcagacc atactgtgca ttggcagaga caagagcagc 1260
tgtgagtcct acggctacaa cgtttgtgac tatcagtgct gggagaactc cgtgggggag 1320
gagctgtaca agctgagtat cttcaacttc ctcctcaccg tggccttcgc cttcctggtc 1380
accctgcctc ggaggctgct ggtggaccgg ttctcaggcc ggttctgggc ctggctggaa 1440
cgggaggagt tcctggtccc caagaatgtg ctggacatcg tggcggggca gacggtcacc 1500
tggatgggcc tcttctactg ccccctgctg cccctgctga atagcgtctt cctcttcctc 1560
accttctaca tcaagaaggt gacggctcat ggctgggggt acaccctcct gaagaactcc 1620
agggcatctt cgcggccctt ccgtgcctcc agctccacct tcttcttcca gctagtgctc 1680
ctcctgggcc tgcttctggc tgcagtgccc ctgggctatg tggtcagcag ccccacttgg 1740
ccatctctcg ccagcatcca ctcctcctgg gactgcggcc tcttcaccaa ctactcagca 1800
ccctggcaag tggtcccgga gctggtggcc cttgggctcc cgcccattgg ccagcgtgcc 1860
ctccactacc tgggctccca cgccttcagc ttccccctcc tcatcatgct cagccttgtc 1920
ctgacggtgt gcgtctccca gacccaggcc aatgccaggg ccatccacag gctccggaag 1980
cagctggtgt ggcaggttca ggagaagtgg cacctggtgg aggacctgtc gcgactgctg 2040
ccggagccag gcccgagcga ctctccgggc cccaagtacc ctgcctccca agcttcgcgc 2100
ccgcagtcct tctgccccgg atgcccatgc cctggctccc cgggccacca ggccccgcgg 2160
ccgggcccct ccgtcgtgga tgccgcggga ctgcgttccc cttgccctgg acagcacggt 2220
gccccggcct ccgcccgcag attccgcttc cccagcggcg cggagctgta acccctaccc 2280
ctgcctcccc gaagcctccc tggggcccct tcaggcctcc ttactccatc ttccagaccc 2340
ctggcgacca ccgcccctct cagtggctcc agggcctccc tcagaggtc 2389

<210> 10
<211> 93
<212> PRT

<213> human

<400> 10
Met Ala Gln Pro Leu Ala Phe Ile Leu Asp Val Pro Glu Thr Pro Gly
1               5                   10                  15

Asp Gln Gly Gln Gly Pro Ser Pro Tyr Asp Glu Ser Glu Val His Asp
            20                  25                  30

Ser Phe Gln Gln Leu Ile Gln Glu Gln Ser Gln Cys Thr Ala Gln Glu
        35                  40                  45

Gly Leu Glu Leu Gln Gln Arg Glu Arg Glu Val Thr Gly Ser Ser Gln
    50                  55                  60

Gln Thr Leu Trp Arg Pro Glu Gly Thr Gln Ser Thr Ala Thr Leu Arg
65                  70                  75                  80

Ile Leu Ala Ser Met Pro Ser Arg Thr Ile Gly Arg Ser
                85                  90

<210> 11
<211> 575
<212> PRT
<213> human

<400> 11
Met Ala Gln Pro Leu Ala Phe Ile Leu Asp Val Pro Glu Thr Pro Gly
1               5                   10                  15

Asp Gln Gly Gln Gly Pro Ser Pro Tyr Asp Glu Ser Glu Val His Asp
            20                  25                  30

Ser Phe Gln Gln Leu Ile Gln Glu Gln Ser Gln Cys Thr Ala Gln Glu
        35                  40                  45

Gly Leu Glu Leu Gln Gln Arg Glu Arg Glu Val Thr Gly Ser Ser Gln
    50                  55                  60

Gln Thr Leu Trp Arg Pro Glu Gly Thr Gln Ser Thr Ala Thr Leu Arg
65                  70                  75                  80

Ile Leu Ala Ser Met Pro Ser Arg Thr Ile Gly Arg Ser Arg Gly Ala
                85                  90                  95

Ile Ile Ser Gln Tyr Tyr Asn Arg Thr Val Gln Leu Arg Cys Arg Ser

            100                 105                 110

Ser Arg Pro Leu Leu Gly Asn Phe Val Arg Ser Ala Arg Pro Ser Leu
        115                 120                 125

Arg Leu Tyr Asp Leu Glu Leu Asp Pro Thr Ala Leu Glu Glu Glu Glu
    130                 135                 140

Lys Gln Ser Leu Leu Val Lys Glu Leu Gln Ser Leu Ala Val Ala Gln
145                 150                 155                 160

Arg Asp His Met Leu Arg Gly Met Pro Leu Ser Leu Ala Glu Lys Arg
                165                 170                 175

Ser Leu Arg Glu Lys Ser Arg Thr Pro Arg Gly Lys Trp Arg Gly Gln
            180                 185                 190

Pro Gly Ser Gly Gly Val Cys Ser Cys Cys Gly Arg Leu Arg Tyr Ala
        195                 200                 205

Cys Val Leu Ala Leu His Ser Leu Gly Leu Ala Leu Leu Ser Ala Leu
    210                 215                 220

Gln Ala Leu Met Pro Trp Arg Tyr Ala Leu Lys Arg Ile Gly Gly Gln
225                 230                 235                 240

Phe Gly Ser Ser Val Leu Ser Tyr Phe Leu Phe Leu Lys Thr Leu Leu
                245                 250                 255

Ala Phe Asn Ala Leu Leu Leu Leu Leu Leu Val Ala Phe Ile Met Gly
            260                 265                 270

Pro Gln Val Ala Phe Pro Pro Ala Leu Pro Gly Pro Ala Pro Val Cys
        275                 280                 285

Thr Gly Leu Glu Leu Leu Thr Gly Ala Gly Cys Phe Thr His Thr Val
    290                 295                 300

Met Tyr Tyr Gly His Tyr Ser Asn Ala Thr Leu Asn Gln Pro Cys Gly
305                 310                 315                 320

Ser Pro Leu Asp Gly Ser Gln Cys Thr Pro Arg Val Gly Gly Leu Pro
                325                 330                 335

Tyr Asn Met Pro Leu Ala Tyr Leu Ser Thr Val Gly Val Ser Phe Phe
            340                 345                 350

Ile Thr Cys Ile Thr Leu Val Tyr Ser Met Ala His Ser Phe Gly Glu

355                 360                 365

Ser Tyr Arg Val Gly Ser Thr Ser Gly Ile His Ala Ile Thr Val Phe
    370                 375                 380

Cys Ser Trp Asp Tyr Lys Val Thr Gln Lys Arg Ala Ser Arg Leu Gln
385                 390                 395                 400

Gln Asp Asn Ile Arg Thr Arg Leu Lys Glu Leu Leu Ala Glu Trp Gln
                405                 410                 415

Leu Arg His Ser Pro Arg Ser Val Cys Gly Arg Leu Arg Gln Ala Ala
            420                 425                 430

Val Leu Gly Leu Val Trp Leu Leu Cys Leu Gly Thr Ala Leu Gly Cys
        435                 440                 445

Ala Val Ala Val His Val Phe Ser Glu Phe Met Ile Gln Ser Pro Glu
    450                 455                 460

Ala Ala Gly Gln Glu Ala Val Leu Leu Val Leu Pro Leu Val Val Gly
465                 470                 475                 480

Leu Leu Asn Leu Gly Ala Pro Tyr Leu Cys Arg Val Leu Ala Ala Leu
                485                 490                 495

Glu Pro His Asp Ser Pro Val Leu Glu Val Tyr Val Ala Ile Cys Arg
            500                 505                 510

Asn Leu Ile Leu Lys Leu Ala Ile Leu Gly Thr Leu Cys Tyr His Trp
        515                 520                 525

Leu Gly Arg Arg Val Gly Val Leu Gln Gly Gln Cys Trp Glu Asp Phe
    530                 535                 540

Val Gly Gln Glu Leu Tyr Arg Phe Leu Val Met Asp Phe Val Leu Met
545                 550                 555                 560

Leu Leu Asp Thr Leu Phe Gly Glu Leu Val Trp Arg Ile Ile Ser
                565                 570                 575

<210> 12
<211> 376
<212> PRT
<213> human

<400> 12

Met Leu Leu Pro Arg Ser Val Ser Ser Glu Arg Ala Pro Gly Val Pro
1               5                   10                  15

Glu Pro Glu Glu Leu Trp Glu Ala Glu Met Glu Arg Leu Arg Gly Ser
            20                  25                  30

Gly Thr Pro Val Arg Gly Leu Pro Tyr Ala Met Met Asp Lys Arg Leu
        35                  40                  45

Ile Trp Gln Leu Arg Glu Pro Ala Gly Val Gln Thr Leu Arg Trp Gln
    50                  55                  60

Arg Trp Gln Arg Arg Arg Gln Thr Val Glu Arg Arg Leu Arg Glu Ala
65                  70                  75                  80

Ala Gln Arg Leu Ala Arg Gly Leu Gly Leu Trp Glu Gly Ala Leu Tyr
                85                  90                  95

Glu Ile Gly Gly Leu Phe Gly Thr Gly Ile Arg Ser Tyr Phe Thr Phe
            100                 105                 110

Leu Arg Phe Leu Leu Leu Leu Asn Leu Leu Ser Leu Leu Leu Thr Ala
        115                 120                 125

Ser Phe Val Leu Leu Pro Leu Val Trp Leu Arg Pro Pro Asp Pro Gly
    130                 135                 140

Pro Thr Leu Asn Leu Thr Leu Gln Cys Pro Gly Ser Arg Gln Ser Pro
145                 150                 155                 160

Pro Gly Val Leu Arg Phe His Asn Gln Leu Trp His Val Leu Thr Gly
                165                 170                 175

Arg Ala Phe Thr Asn Thr Tyr Leu Phe Tyr Gly Ala Tyr Arg Val Gly
            180                 185                 190

Pro Glu Ser Ser Ser Val Tyr Ser Ile Arg Leu Ala Tyr Leu Leu Ser
        195                 200                 205

Pro Leu Ala Cys Leu Leu Leu Cys Phe Cys Gly Thr Leu Arg Arg Met
    210                 215                 220

Val Lys Gly Leu Pro Gln Lys Thr Leu Leu Gly Gln Gly Tyr Gln Ala
225                 230                 235                 240

Pro Leu Ser Ala Lys Val Phe Ser Ser Trp Asp Phe Cys Ile Arg Val
                245                 250                 255

Gln Glu Ala Ala Thr Ile Lys Lys His Glu Ile Ser Asn Glu Phe Lys
            260                 265                 270

Val Glu Leu Glu Glu Gly Arg Arg Phe Gln Leu Met Gln Gln Gln Thr
        275                 280                 285

Arg Ala Gln Thr Ala Cys Arg Leu Leu Ser Tyr Leu Arg Val Asn Val
    290                 295                 300

Leu Asn Gly Leu Leu Val Val Gly Ala Ile Ser Ala Ile Phe Trp Ala
305                 310                 315                 320

Thr Lys Tyr Ser Gln Asp Asn Lys Glu Val Ser Gly Asn Cys Ile His
                325                 330                 335

Leu Ile Leu Ala Arg Thr Ala Gly Glu Ser Leu Phe Leu Leu Leu Gln
            340                 345                 350

Tyr Leu Pro Pro Gly Val Ile Ala Leu Val Asn Phe Leu Gly Pro Leu
        355                 360                 365

Leu Phe Thr Phe Leu Val Gln Leu
    370                 375

<210> 13
<211> 24
<212> DNA
<213> Artificial Sequence

<220>
<223> Description of Artificial Sequence: primer

<400> 13
aggcgcaatg agctgcatta acct                                          24

<210> 14
<211> 20
<212> DNA
<213> Artificial Sequence

<220>
<223> Description of Artificial Sequence: primer

<400> 14

gcgacggcga catggagagc                                                    20


<210> 15
<211> 28
<212> DNA
<213> Artificial Sequence

<220>
<223> Description of Artificial Sequence: primer

<400> 15
agactagcgg gcattggcca gagacatg                                            28


<210> 16
<211> 22
<212> DNA
<213> Artificial Sequence

<220>
<223> Description of Artificial Sequence: primer

<400> 16
ctggcccgcc gagatgctgc tg                                                  22


<210> 17
<211> 24
<212> DNA
<213> Artificial Sequence

<220>
<223> Description of Artificial Sequence: primer

<400> 17
aagagtgagc catgctgtac acca                                                24


<210> 18
<211> 23
<212> DNA
<213> Artificial Sequence

<220>
<223> Description of Artificial Sequence: primer

<400> 18

gctacagcag aggcgtggac cct 23

<210> 19
<211> 20
<212> DNA
<213> Artificial Sequence

<220>
<223> Description of Artificial Sequence: primer

<400> 19
cgtctccaaa cagcatccca 20

<210> 20
<211> 24
<212> DNA
<213> Artificial Sequence

<220>
<223> Description of Artificial Sequence: primer

<400> 20
gaggcctgaa ggggccccag ggag 24

<210> 21
<211> 24
<212> DNA
<213> Artificial Sequence

<220>
<223> Description of Artificial Sequence: primer

<400> 21
ccagctccag cctaggcgca gctg 24

<210> 22
<211> 285
<212> DNA
<213> human

<400> 22
gaatgatgga attaatagac aaggacctct ctctccacac acacacacac acacacacac 60
acacacacac acacaccaca cactatatac agtggttctc ttatccatgg agcatatgtt 120
ccaaagtccc cagtagatgc ctgaaactgc aaatagtcct gaaccttata tatacctggt 180

ttttcttata catacatgcc tataatactg tttaatgtat aaattaggct cagtaacaga 240
ttaacaataa taaataatag taaaatagaa catataacaa taagc 285

<210> 23
<211> 246
<212> DNA
<213> human

<400> 23
gcgatcagtt aatggcactg gatgcaatga ggtgtggggt gtggggtggg tgctcctgag 60
agtaaggagg gcgtgtgaga gagtgtgtgt gagagtgtgt gtgtgtgtgt gtgtgtgcac 120
gcagcattag acacatgctc tctgcaatct cctgctgttg tcctcagaca ttccaggagc 180
ttccctaatc tcctgatttt tgggagaaac tcccgccccc ctggcccagt ggacctgaga 240
gagtga 246

<210> 24
<211> 226
<212> DNA
<213> human

<400> 24
ttacaggtgc tgagtgtaaa tagccttgac cctgaaggag gactgcattt tatagccctc 60
aaaatagccc cacactcacc ctttgcacac ctcacaatca agcctatgga ccccacacac 120
acacacacac acacacaccc cacatgcaca ggcacacatg tcatctcatc tacaacagaa 180
cctgctgtcc acaggcattt tctgcagtgc catgcctcag gcctca 226

<210> 25
<211> 218
<212> DNA
<213> human

<400> 25
cacatcatac aagaagctag gaaccatatt ctattccaga ccattcatat cctctctctc 60
tctgcctctc tctctctctc tccttacaca cacacacaca cacacacaca cacacgccag 120
ccataagtca aaacaagggc tacagcgaac tctaaagatt cacaataaca cagctatttc 180
cacacctgtg caacattaac ctactatggt cttcacca 218

<210> 26
<211> 221
<212> DNA
<213> human

<400> 26
agagttaact gttctggatc atccatttct tcttcttctt cttctttctt cttcttcttc 60

ttcttcttct tcttcttctt cttcttcttc ttcttcttct tcttcttctt cttccttctt 120
ccttcttcct tcttcttcct tcttccttct tcctccttct tccttcttct tctttcttct 180
tcttccttct tcttgaattt ttctgtctgg tttagtacca g 221

<210> 27
<211> 144
<212> DNA
<213> human

<400> 27
actgtaaaca tgtgggatag aaacaacccg gtcatccaga gcagtaccac gaccaatacc 60
accaccacca ccaccactac cacgagcaac accacacaca gggtcgagac gccgcccccg 120
caccaggccg gtactcagct gaat 144

<210> 28
<211> 198
<212> DNA
<213> human

<400> 28
ggttgacatc agtacacaga aacaggctct aggaaaaatt tctaagttca tagcctacgt 60
gtgtgtatgt gtgcgtgttg aaacagaagt gtgtgtgtgt gtgcatgtgt ggcgcgtgct 120
cctgcacggc gtgcagtttt ctgtgtttgt gtgtttgaaa gcgtgtgagg atttaactgt 180
gggttttccc ttgtattc 198

<210> 29
<211> 148
<212> DNA
<213> human

<400> 29
agtacacaga aacaggctct aggaaaaatt tctaagttca tagcctacgt gtgtgtatgt 60
gtgcgtgttg aaacagaagt gtgtgtgtgt gtgcatgtgt ggcgcgtgct cctgcacggc 120
gtgcagtttt ctgtgtttgt gtgtttga 148

<210> 30
<211> 189
<212> DNA
<213> human

<400> 30
ccacgggtcc gccttcccac ccgaagttga gggaccccaa gatgtacccc ctccctgtaa 60
ggtgggcaat acctccaaaa ccaggttttg tgtgtgtgtg tgtgtggcgg ggagggggcc 120
tgttcttgga cagccagccc tgaaagctgg agggggtggc atgagggggc aggcagaggt 180

gggcctgct                                                          189

<210> 31
<211> 191
<212> DNA
<213> human

<400> 31
tggggtggag ctccttcctc ttctccgggg accccttgtc cctcccctcc cctcccctcc 60
cctcccctcc cctcccctcc ccttccctcc ccttcccttc cctccccttc ccttccccta 120
gaaggaccag cacagcctcc tacagctccc gcccggggtg ctcctccctt gaattcagtc 180
caggaggaag t                                                       191